# EUROPÄISCHE PATENTANMELDUNG

(11) **EP 2 060 636 A1**
(43) Veröffentlichungstag der Anmeldung: **20.05.2009**
(21) Anmeldenummer: 08167366.7
(22) Anmeldetag: 23.10.2008
(51) Int. Cl.: C12P 13/08

(54) **Verfahren zur Herstellung von L-Aminosäuren unter Verwendung von verbesserten Stämmen der Familie Enterobacteriaceae**

(30) Priorität: 14.11.2007 DE 102007054250
(71) Anmelder: Evonik Degussa GmbH, 45128 Essen (DE)
(72) Erfinder: Rieping, Mechthild, Dr., 33619 Bielefeld (DE); Jerrentrup, Silke, 33607 Bielefeld (DE)

(57) **Zusammenfassung**

Die Erfindung betrifft ein Verfahren zur Herstellung von L-Aminosäuren durch Fermentation von rekombinanten Mikroorganismen der Familie Enterobacteriaceae, dadurch gekennzeichnet, dass man
a) die die gewünschte L-Aminosäure produzierenden Mikroorganismen, in denen man das glgS-Gen oder für dessen Genprodukt kodierende Nukleotidsequenzen oder Allele verstärkt, insbesondere überexprimiert, in einem Medium kultiviert unter Bedingungen, bei denen die gewünschte L-Aminosäure im Medium oder in den Zellen angereichert wird, und
b) die gewünschte L-Aminosäure isoliert, wobei gegebenenfalls weitere Bestandteile der Fermentationsbrühe und/oder die Biomasse in ihrer Gesamtheit oder Anteilen (≥ 0 bis 100 %) im isolierten Produkt verbleiben oder vollständig entfernt werden.

SEQUENZPROTOKOLL

<110>  Evonik Degussa GmbH

<120>  Verfahren zur Herstellung von L-Aminosäuren unter Verwendung von
       verbesserten Stämmen der Familie Enterobacteriaceae

<130>  200700660 AL

<160>  16

<170>  PatentIn version 3.3

<210>  1
<211>  201
<212>  DNA
<213>  Escherichia coli


<220>
<221>  CDS
<222>  (1)..(201)
<223>  glgS-Kodierregion

<400>  1
atg gat cat agt ctt aat tct tta aat aat ttc gat ttc ctg gcg cgt       48
Met Asp His Ser Leu Asn Ser Leu Asn Asn Phe Asp Phe Leu Ala Arg
1               5                   10                  15

agt ttt gcc aga atg cac gca gaa ggt cgc ccg gtc gat att ctg gcc       96
Ser Phe Ala Arg Met His Ala Glu Gly Arg Pro Val Asp Ile Leu Ala
            20                  25                  30

gtt act ggt aac atg gat gaa gaa cat aga acc tgg ttt tgc gca cgt      144
Val Thr Gly Asn Met Asp Glu Glu His Arg Thr Trp Phe Cys Ala Arg
        35                  40                  45

tat gcc tgg tat tgt caa cag atg atg cag gca aga gag ctg gag tta      192
Tyr Ala Trp Tyr Cys Gln Gln Met Met Gln Ala Arg Glu Leu Glu Leu
    50                  55                  60

gag cac tga                                                          201
Glu His
65


<210>  2
<211>  66
<212>  PRT
<213>  Escherichia coli

<400>  2

Met Asp His Ser Leu Asn Ser Leu Asn Asn Phe Asp Phe Leu Ala Arg
1               5                   10                  15


Ser Phe Ala Arg Met His Ala Glu Gly Arg Pro Val Asp Ile Leu Ala
            20                  25                  30


Val Thr Gly Asn Met Asp Glu Glu His Arg Thr Trp Phe Cys Ala Arg
        35                  40                  45


Tyr Ala Trp Tyr Cys Gln Gln Met Met Gln Ala Arg Glu Leu Glu Leu
    50                  55                  60

Glu His
65

<210> 3
<211> 201
<212> DNA
<213> Shigella flexneri

<220>
<221> CDS
<222> (1)..(201)
<223> glgS-Kodierregion

<400> 3
atg gag cat agt ctt aat tct ttg aat aat ttc gat ttc ctg gcg cgt 48
Met Glu His Ser Leu Asn Ser Leu Asn Asn Phe Asp Phe Leu Ala Arg
1               5                   10                  15

agt ttt gcc aga atg cac gca gaa ggt cgc ccg gtc gat att ctg gct 96
Ser Phe Ala Arg Met His Ala Glu Gly Arg Pro Val Asp Ile Leu Ala
            20                  25                  30

gtt act ggt aac atg gat gaa gaa cat aga act tgg ttt tgc gca cgt 144
Val Thr Gly Asn Met Asp Glu Glu His Arg Thr Trp Phe Cys Ala Arg
        35                  40                  45

tat gcc tgg tat tgc caa cag atg atg cag gca aga gag ctg gag tta 192
Tyr Ala Trp Tyr Cys Gln Gln Met Met Gln Ala Arg Glu Leu Glu Leu
    50                  55                  60

gag cac tga 201
Glu His
65

<210> 4
<211> 66
<212> PRT
<213> Shigella flexneri

<400> 4

Met Glu His Ser Leu Asn Ser Leu Asn Asn Phe Asp Phe Leu Ala Arg
1               5                   10                  15

Ser Phe Ala Arg Met His Ala Glu Gly Arg Pro Val Asp Ile Leu Ala
            20                  25                  30

Val Thr Gly Asn Met Asp Glu Glu His Arg Thr Trp Phe Cys Ala Arg
        35                  40                  45

Tyr Ala Trp Tyr Cys Gln Gln Met Met Gln Ala Arg Glu Leu Glu Leu
    50                  55                  60

Glu His
65

<210> 5
<211> 204
<212> DNA
<213> Salmonella enterica

<220>
<221>  CDS
<222>  (1)..(204)
<223>  glgS-Kodierregion

<400>  5
atg aac aac aat aac gtc tat tca tta aat aat ttc gat ttt ctg gcg        48
Met Asn Asn Asn Asn Val Tyr Ser Leu Asn Asn Phe Asp Phe Leu Ala
1               5                   10                  15

cgc agc ttt gcc aga atg cag gcg gag ggg cgt cct gtt gat atc cag        96
Arg Ser Phe Ala Arg Met Gln Ala Glu Gly Arg Pro Val Asp Ile Gln
            20                  25                  30

gcg gta acc ggc aat atg gac gag gag cac cgc gac tgg ttt tgc aag       144
Ala Val Thr Gly Asn Met Asp Glu Glu His Arg Asp Trp Phe Cys Lys
        35                  40                  45

cgt tac gcg ctt tac tgc cag cag gcg act cag gcg aaa aag tta gaa       192
Arg Tyr Ala Leu Tyr Cys Gln Gln Ala Thr Gln Ala Lys Lys Leu Glu
    50                  55                  60

tta gaa cac taa                                                       204
Leu Glu His
65


<210>  6
<211>  67
<212>  PRT
<213>  Salmonella enterica

<400>  6

Met Asn Asn Asn Asn Val Tyr Ser Leu Asn Asn Phe Asp Phe Leu Ala
1               5                   10                  15


Arg Ser Phe Ala Arg Met Gln Ala Glu Gly Arg Pro Val Asp Ile Gln
            20                  25                  30


Ala Val Thr Gly Asn Met Asp Glu Glu His Arg Asp Trp Phe Cys Lys
        35                  40                  45


Arg Tyr Ala Leu Tyr Cys Gln Gln Ala Thr Gln Ala Lys Lys Leu Glu
    50                  55                  60


Leu Glu His
65


<210>  7
<211>  2463
<212>  DNA
<213>  Escherichia coli


<220>
<221>  CDS
<222>  (1)..(2463)
<223>  thrA-Kodierregion

<400>  7
atg cga gtg ttg aag ttc ggc ggt aca tca gtg gca aat gca gaa cgt        48
Met Arg Val Leu Lys Phe Gly Gly Thr Ser Val Ala Asn Ala Glu Arg

1                   5                   10                  15

ttt ctg cgt gtt gcc gat att ctg gaa agc aat gcc agg cag ggg cag        96
Phe Leu Arg Val Ala Asp Ile Leu Glu Ser Asn Ala Arg Gln Gly Gln
            20                  25                  30

gtg gcc acc gtc ctc tct gcc ccc gcc aaa atc acc aac cac ctg gtg       144
Val Ala Thr Val Leu Ser Ala Pro Ala Lys Ile Thr Asn His Leu Val
        35                  40                  45

gcg atg att gaa aaa acc att agc ggc cag gat gct tta ccc aat atc       192
Ala Met Ile Glu Lys Thr Ile Ser Gly Gln Asp Ala Leu Pro Asn Ile
    50                  55                  60

agc gat gcc gaa cgt att ttt gcc gaa ctt ttg acg gga ctc gcc gcc       240
Ser Asp Ala Glu Arg Ile Phe Ala Glu Leu Leu Thr Gly Leu Ala Ala
65                  70                  75                  80

gcc cag ccg ggg ttc ccg ctg gcg caa ttg aaa act ttc gtc gat cag       288
Ala Gln Pro Gly Phe Pro Leu Ala Gln Leu Lys Thr Phe Val Asp Gln
                85                  90                  95

gaa ttt gcc caa ata aaa cat gtc ctg cat ggc att agt ttg ttg ggg       336
Glu Phe Ala Gln Ile Lys His Val Leu His Gly Ile Ser Leu Leu Gly
            100                 105                 110

cag tgc ccg gat agc atc aac gct gcg ctg att tgc cgt ggc gag aaa       384
Gln Cys Pro Asp Ser Ile Asn Ala Ala Leu Ile Cys Arg Gly Glu Lys
        115                 120                 125

atg tcg atc gcc att atg gcc ggc gta tta gaa gcg cgc ggt cac aac       432
Met Ser Ile Ala Ile Met Ala Gly Val Leu Glu Ala Arg Gly His Asn
    130                 135                 140

gtt act gtt atc gat ccg gtc gaa aaa ctg ctg gca gtg ggg cat tac       480
Val Thr Val Ile Asp Pro Val Glu Lys Leu Leu Ala Val Gly His Tyr
145                 150                 155                 160

ctc gaa tct acc gtc gat att gct gag tcc acc cgc cgt att gcg gca       528
Leu Glu Ser Thr Val Asp Ile Ala Glu Ser Thr Arg Arg Ile Ala Ala
                165                 170                 175

agc cgc att ccg gct gat cac atg gtg ctg atg gca ggt ttc acc gcc       576
Ser Arg Ile Pro Ala Asp His Met Val Leu Met Ala Gly Phe Thr Ala
            180                 185                 190

ggt aat gaa aaa ggc gaa ctg gtg gtg ctt gga cgc aac ggt tcc gac       624
Gly Asn Glu Lys Gly Glu Leu Val Val Leu Gly Arg Asn Gly Ser Asp
        195                 200                 205

tac tct gct gcg gtg ctg gct gcc tgt tta cgc gcc gat tgt tgc gag       672
Tyr Ser Ala Ala Val Leu Ala Ala Cys Leu Arg Ala Asp Cys Cys Glu
    210                 215                 220

att tgg acg gac gtt gac ggg gtc tat acc tgc gac ccg cgt cag gtg       720
Ile Trp Thr Asp Val Asp Gly Val Tyr Thr Cys Asp Pro Arg Gln Val
225                 230                 235                 240

ccc gat gcg agg ttg ttg aag tcg atg tcc tac cag gaa gcg atg gag       768
Pro Asp Ala Arg Leu Leu Lys Ser Met Ser Tyr Gln Glu Ala Met Glu
                245                 250                 255

ctt tcc tac ttc ggc gct aaa gtt ctt cac ccc cgc acc att acc ccc       816
Leu Ser Tyr Phe Gly Ala Lys Val Leu His Pro Arg Thr Ile Thr Pro
            260                 265                 270

atc gcc cag ttc cag atc cct tgc ctg att aaa aat acc gga aat cct       864
Ile Ala Gln Phe Gln Ile Pro Cys Leu Ile Lys Asn Thr Gly Asn Pro
        275                 280                 285

caa gca cca ggt acg ctc att ggt gcc agc cgt gat gaa gac gaa tta 912
Gln Ala Pro Gly Thr Leu Ile Gly Ala Ser Arg Asp Glu Asp Glu Leu
    290                 295                 300

ccg gtc aag ggc att tcc aat ctg aat aac atg gca atg ttc agc gtt 960
Pro Val Lys Gly Ile Ser Asn Leu Asn Asn Met Ala Met Phe Ser Val
305                 310                 315                 320

tct ggt ccg ggg atg aaa ggg atg gtc ggc atg gcg gcg cgc gtc ttt 1008
Ser Gly Pro Gly Met Lys Gly Met Val Gly Met Ala Ala Arg Val Phe
                325                 330                 335

gca gcg atg tca cgc gcc cgt att tcc gtg gtg ctg att acg caa tca 1056
Ala Ala Met Ser Arg Ala Arg Ile Ser Val Val Leu Ile Thr Gln Ser
            340                 345                 350

tct tcc gaa tac agc atc agt ttc tgc gtt cca caa agc gac tgt gtg 1104
Ser Ser Glu Tyr Ser Ile Ser Phe Cys Val Pro Gln Ser Asp Cys Val
        355                 360                 365

cga gct gaa cgg gca atg cag gaa gag ttc tac ctg gaa ctg aaa gaa 1152
Arg Ala Glu Arg Ala Met Gln Glu Glu Phe Tyr Leu Glu Leu Lys Glu
    370                 375                 380

ggc tta ctg gag ccg ctg gca gtg acg gaa cgg ctg gcc att atc tcg 1200
Gly Leu Leu Glu Pro Leu Ala Val Thr Glu Arg Leu Ala Ile Ile Ser
385                 390                 395                 400

gtg gta ggt gat ggt atg cgc acc ttg cgt ggg atc tcg gcg aaa ttc 1248
Val Val Gly Asp Gly Met Arg Thr Leu Arg Gly Ile Ser Ala Lys Phe
                405                 410                 415

ttt gcc gca ctg gcc cgc gcc aat atc aac att gtc gcc att gct cag 1296
Phe Ala Ala Leu Ala Arg Ala Asn Ile Asn Ile Val Ala Ile Ala Gln
            420                 425                 430

gga tct tct gaa cgc tca atc tct gtc gtg gta aat aac gat gat gcg 1344
Gly Ser Ser Glu Arg Ser Ile Ser Val Val Val Asn Asn Asp Asp Ala
        435                 440                 445

acc act ggc gtg cgc gtt act cat cag atg ctg ttc aat acc gat cag 1392
Thr Thr Gly Val Arg Val Thr His Gln Met Leu Phe Asn Thr Asp Gln
    450                 455                 460

gtt atc gaa gtg ttt gtg att ggc gtc ggt ggc gtt ggc ggt gcg ctg 1440
Val Ile Glu Val Phe Val Ile Gly Val Gly Gly Val Gly Gly Ala Leu
465                 470                 475                 480

ctg gag caa ctg aag cgt cag caa agc tgg ctg aag aat aaa cat atc 1488
Leu Glu Gln Leu Lys Arg Gln Gln Ser Trp Leu Lys Asn Lys His Ile
                485                 490                 495

gac tta cgt gtc tgc ggt gtt gcc aac tcg aag gct ctg ctc acc aat 1536
Asp Leu Arg Val Cys Gly Val Ala Asn Ser Lys Ala Leu Leu Thr Asn
            500                 505                 510

gta cat ggc ctt aat ctg gaa aac tgg cag gaa gaa ctg gcg caa gcc 1584
Val His Gly Leu Asn Leu Glu Asn Trp Gln Glu Glu Leu Ala Gln Ala
        515                 520                 525

aaa gag ccg ttt aat ctc ggg cgc tta att cgc ctc gtg aaa gaa tat 1632
Lys Glu Pro Phe Asn Leu Gly Arg Leu Ile Arg Leu Val Lys Glu Tyr
    530                 535                 540

cat ctg ctg aac ccg gtc att gtt gac tgc act tcc agc cag gca gtg 1680
His Leu Leu Asn Pro Val Ile Val Asp Cys Thr Ser Ser Gln Ala Val
545                 550                 555                 560

gcg gat caa tat gcc gac ttc ctg cgc gaa ggt ttc cac gtt gtc acg 1728
Ala Asp Gln Tyr Ala Asp Phe Leu Arg Glu Gly Phe His Val Val Thr
                565                 570                 575

ccg aac aaa aag gcc aac acc tcg tcg atg gat tac tac cat cag ttg 1776
Pro Asn Lys Lys Ala Asn Thr Ser Ser Met Asp Tyr Tyr His Gln Leu
            580                 585                 590

cgt tat gcg gcg gaa aaa tcg cgg cgt aaa ttc ctc tat gac acc aac 1824
Arg Tyr Ala Ala Glu Lys Ser Arg Arg Lys Phe Leu Tyr Asp Thr Asn
        595                 600                 605

gtt ggg gct gga tta ccg gtt att gag aac ctg caa aat ctg ctc aat 1872
Val Gly Ala Gly Leu Pro Val Ile Glu Asn Leu Gln Asn Leu Leu Asn
    610                 615                 620

gca ggt gat gaa ttg atg aag ttc tcc ggc att ctt tct ggt tcg ctt 1920
Ala Gly Asp Glu Leu Met Lys Phe Ser Gly Ile Leu Ser Gly Ser Leu
625                 630                 635                 640

tct tat atc ttc ggc aag tta gac gaa ggc atg agt ttc tcc gag gcg 1968
Ser Tyr Ile Phe Gly Lys Leu Asp Glu Gly Met Ser Phe Ser Glu Ala
                645                 650                 655

acc acg ctg gcg cgg gaa atg ggt tat acc gaa ccg gac ccg cga gat 2016
Thr Thr Leu Ala Arg Glu Met Gly Tyr Thr Glu Pro Asp Pro Arg Asp
            660                 665                 670

gat ctt tct ggt atg gat gtg gcg cgt aaa cta ttg att ctc gct cgt 2064
Asp Leu Ser Gly Met Asp Val Ala Arg Lys Leu Leu Ile Leu Ala Arg
        675                 680                 685

gaa acg gga cgt gaa ctg gag ctg gcg gat att gaa att gaa cct gtg 2112
Glu Thr Gly Arg Glu Leu Glu Leu Ala Asp Ile Glu Ile Glu Pro Val
    690                 695                 700

ctg ccc gca gag ttt aac gcc gag ggt gat gtt gcc gct ttt atg gcg 2160
Leu Pro Ala Glu Phe Asn Ala Glu Gly Asp Val Ala Ala Phe Met Ala
705                 710                 715                 720

aat ctg tca caa ctc gac gat ctc ttt gcc gcg cgc gtg gcg aag gcc 2208
Asn Leu Ser Gln Leu Asp Asp Leu Phe Ala Ala Arg Val Ala Lys Ala
                725                 730                 735

cgt gat gaa gga aaa gtt ttg cgc tat gtt ggc aat att gat gaa gat 2256
Arg Asp Glu Gly Lys Val Leu Arg Tyr Val Gly Asn Ile Asp Glu Asp
            740                 745                 750

ggc gtc tgc cgc gtg aag att gcc gaa gtg gat ggt aat gat ccg ctg 2304
Gly Val Cys Arg Val Lys Ile Ala Glu Val Asp Gly Asn Asp Pro Leu
        755                 760                 765

ttc aaa gtg aaa aat ggc gaa aac gcc ctg gcc ttc tat agc cac tat 2352
Phe Lys Val Lys Asn Gly Glu Asn Ala Leu Ala Phe Tyr Ser His Tyr
    770                 775                 780

tat cag ccg ctg ccg ttg gta ctg cgc gga tat ggt gcg ggc aat gac 2400
Tyr Gln Pro Leu Pro Leu Val Leu Arg Gly Tyr Gly Ala Gly Asn Asp
785                 790                 795                 800

gtt aca gct gcc ggt gtc ttt gct gat ctg cta cgt acc ctc tca tgg 2448
Val Thr Ala Ala Gly Val Phe Ala Asp Leu Leu Arg Thr Leu Ser Trp
                805                 810                 815

aag tta gga gtc tga 2463
Lys Leu Gly Val
            820

<210>  8
<211>  820
<212>  PRT
<213>  Escherichia coli

<400>  8

Met Arg Val Leu Lys Phe Gly Gly Thr Ser Val Ala Asn Ala Glu Arg
1               5                   10                  15

Phe Leu Arg Val Ala Asp Ile Leu Glu Ser Asn Ala Arg Gln Gly Gln
            20                  25                  30

Val Ala Thr Val Leu Ser Ala Pro Ala Lys Ile Thr Asn His Leu Val
        35                  40                  45

Ala Met Ile Glu Lys Thr Ile Ser Gly Gln Asp Ala Leu Pro Asn Ile
    50                  55                  60

Ser Asp Ala Glu Arg Ile Phe Ala Glu Leu Leu Thr Gly Leu Ala Ala
65                  70                  75                  80

Ala Gln Pro Gly Phe Pro Leu Ala Gln Leu Lys Thr Phe Val Asp Gln
                85                  90                  95

Glu Phe Ala Gln Ile Lys His Val Leu His Gly Ile Ser Leu Leu Gly
            100                 105                 110

Gln Cys Pro Asp Ser Ile Asn Ala Ala Leu Ile Cys Arg Gly Glu Lys
        115                 120                 125

Met Ser Ile Ala Ile Met Ala Gly Val Leu Glu Ala Arg Gly His Asn
    130                 135                 140

Val Thr Val Ile Asp Pro Val Glu Lys Leu Leu Ala Val Gly His Tyr
145                 150                 155                 160

Leu Glu Ser Thr Val Asp Ile Ala Glu Ser Thr Arg Arg Ile Ala Ala
                165                 170                 175

Ser Arg Ile Pro Ala Asp His Met Val Leu Met Ala Gly Phe Thr Ala
            180                 185                 190

Gly Asn Glu Lys Gly Glu Leu Val Val Leu Gly Arg Asn Gly Ser Asp
        195                 200                 205

Tyr Ser Ala Ala Val Leu Ala Ala Cys Leu Arg Ala Asp Cys Cys Glu
    210                 215                 220

Ile Trp Thr Asp Val Asp Gly Val Tyr Thr Cys Asp Pro Arg Gln Val
225                 230                 235                 240

Pro Asp Ala Arg Leu Leu Lys Ser Met Ser Tyr Gln Glu Ala Met Glu
                245                 250                 255

Leu Ser Tyr Phe Gly Ala Lys Val Leu His Pro Arg Thr Ile Thr Pro
            260                 265                 270

Ile Ala Gln Phe Gln Ile Pro Cys Leu Ile Lys Asn Thr Gly Asn Pro
        275                 280                 285

Gln Ala Pro Gly Thr Leu Ile Gly Ala Ser Arg Asp Glu Asp Glu Leu
    290                 295                 300

Pro Val Lys Gly Ile Ser Asn Leu Asn Asn Met Ala Met Phe Ser Val
305                 310                 315                 320

Ser Gly Pro Gly Met Lys Gly Met Val Gly Met Ala Ala Arg Val Phe
                325                 330                 335

Ala Ala Met Ser Arg Ala Arg Ile Ser Val Val Leu Ile Thr Gln Ser
            340                 345                 350

Ser Ser Glu Tyr Ser Ile Ser Phe Cys Val Pro Gln Ser Asp Cys Val
        355                 360                 365

Arg Ala Glu Arg Ala Met Gln Glu Glu Phe Tyr Leu Glu Leu Lys Glu
    370                 375                 380

Gly Leu Leu Glu Pro Leu Ala Val Thr Glu Arg Leu Ala Ile Ile Ser
385                 390                 395                 400

Val Val Gly Asp Gly Met Arg Thr Leu Arg Gly Ile Ser Ala Lys Phe
                405                 410                 415

Phe Ala Ala Leu Ala Arg Ala Asn Ile Asn Ile Val Ala Ile Ala Gln
            420                 425                 430

Gly Ser Ser Glu Arg Ser Ile Ser Val Val Val Asn Asn Asp Asp Ala
        435                 440                 445

Thr Thr Gly Val Arg Val Thr His Gln Met Leu Phe Asn Thr Asp Gln
    450                 455                 460

Val Ile Glu Val Phe Val Ile Gly Val Gly Gly Val Gly Gly Ala Leu
465                 470                 475                 480

Leu Glu Gln Leu Lys Arg Gln Gln Ser Trp Leu Lys Asn Lys His Ile
                485                 490                 495

Asp Leu Arg Val Cys Gly Val Ala Asn Ser Lys Ala Leu Leu Thr Asn
            500                 505                 510

Val His Gly Leu Asn Leu Glu Asn Trp Gln Glu Glu Leu Ala Gln Ala
        515                 520                 525

Lys Glu Pro Phe Asn Leu Gly Arg Leu Ile Arg Leu Val Lys Glu Tyr
    530                 535                 540

His Leu Leu Asn Pro Val Ile Val Asp Cys Thr Ser Ser Gln Ala Val
545                 550                 555                 560

Ala Asp Gln Tyr Ala Asp Phe Leu Arg Glu Gly Phe His Val Val Thr
                565                 570                 575

Pro Asn Lys Lys Ala Asn Thr Ser Ser Met Asp Tyr Tyr His Gln Leu
            580                 585                 590

Arg Tyr Ala Ala Glu Lys Ser Arg Arg Lys Phe Leu Tyr Asp Thr Asn
        595                 600                 605

Val Gly Ala Gly Leu Pro Val Ile Glu Asn Leu Gln Asn Leu Leu Asn
    610                 615                 620

Ala Gly Asp Glu Leu Met Lys Phe Ser Gly Ile Leu Ser Gly Ser Leu
625                 630                 635                 640

Ser Tyr Ile Phe Gly Lys Leu Asp Glu Gly Met Ser Phe Ser Glu Ala
                645                 650                 655

Thr Thr Leu Ala Arg Glu Met Gly Tyr Thr Glu Pro Asp Pro Arg Asp
            660                 665                 670

Asp Leu Ser Gly Met Asp Val Ala Arg Lys Leu Leu Ile Leu Ala Arg
        675                 680                 685

Glu Thr Gly Arg Glu Leu Glu Leu Ala Asp Ile Glu Ile Glu Pro Val
    690                 695                 700

Leu Pro Ala Glu Phe Asn Ala Glu Gly Asp Val Ala Ala Phe Met Ala
705                 710                 715                 720

Asn Leu Ser Gln Leu Asp Asp Leu Phe Ala Ala Arg Val Ala Lys Ala
                725                 730                 735

Arg Asp Glu Gly Lys Val Leu Arg Tyr Val Gly Asn Ile Asp Glu Asp
            740                 745                 750

Gly Val Cys Arg Val Lys Ile Ala Glu Val Asp Gly Asn Asp Pro Leu
        755                 760                 765

Phe Lys Val Lys Asn Gly Glu Asn Ala Leu Ala Phe Tyr Ser His Tyr
    770                 775                 780

Tyr Gln Pro Leu Pro Leu Val Leu Arg Gly Tyr Gly Ala Gly Asn Asp
785                 790                 795                 800

Val Thr Ala Ala Gly Val Phe Ala Asp Leu Leu Arg Thr Leu Ser Trp
                805                 810                 815

Lys Leu Gly Val
            820

<210>  9
<211>  2460
<212>  DNA
<213>  Serratia marcescens

<220>
<221>  CDS
<222>  (1)..(2460)
<223>  thrA-Kodierregion

<400>  9
atg cga gtg ctg aaa ttt ggc gga acc tcg gta gcg aat gcg gaa cgt        48
Met Arg Val Leu Lys Phe Gly Gly Thr Ser Val Ala Asn Ala Glu Arg
1               5                   10                  15

ttt ctg cgc gtc gcc gac atc atg gag agt aac gcg cgt cag gga cag        96
Phe Leu Arg Val Ala Asp Ile Met Glu Ser Asn Ala Arg Gln Gly Gln
            20                  25                  30

gta gcc acg gtc ttg tcc gcc ccc gca aag atc acc aac cat ctg gtg       144
Val Ala Thr Val Leu Ser Ala Pro Ala Lys Ile Thr Asn His Leu Val
        35                  40                  45

gcg atg atc gac aaa acg gtg gcg ggc cag gac att ctg ccg aat atg       192
Ala Met Ile Asp Lys Thr Val Ala Gly Gln Asp Ile Leu Pro Asn Met
    50                  55                  60

agc gac gcc gag cgg atc ttt gcc gac ctg ctg agc gga ctg gcg cag       240
Ser Asp Ala Glu Arg Ile Phe Ala Asp Leu Leu Ser Gly Leu Ala Gln
65                  70                  75                  80

gcg ctg ccg ggc ttt gaa tac gat cgt ttg aaa ggc gtg gtc gat cag       288
Ala Leu Pro Gly Phe Glu Tyr Asp Arg Leu Lys Gly Val Val Asp Gln
                85                  90                  95

gaa ttc gcg cag ctc aaa cag gtg ctg cat ggc gtc tcg ctg ctg ggg       336
Glu Phe Ala Gln Leu Lys Gln Val Leu His Gly Val Ser Leu Leu Gly
            100                 105                 110

cag tgc ccg gac agc gtg aac gcg gcg atc atc tgc cgt ggc gaa aag       384
Gln Cys Pro Asp Ser Val Asn Ala Ala Ile Ile Cys Arg Gly Glu Lys
        115                 120                 125

ctc tcc atc gcc atc atg gaa ggg gtg ttc cgc gcc aag ggt tat ccg       432
Leu Ser Ile Ala Ile Met Glu Gly Val Phe Arg Ala Lys Gly Tyr Pro
    130                 135                 140

gtc acg gtg atc aac ccg gtg gag aaa ctg ctg gcg cag ggc cac tac       480
Val Thr Val Ile Asn Pro Val Glu Lys Leu Leu Ala Gln Gly His Tyr
145                 150                 155                 160

ctg gag tcc acc gtg gac atc gcc gag tct acg ctg cgc atc gcc gcc       528
Leu Glu Ser Thr Val Asp Ile Ala Glu Ser Thr Leu Arg Ile Ala Ala
                165                 170                 175

gcg gcg atc ccg gcc gat cac atc gtg ctg atg gcc ggt ttc acc gcc       576
Ala Ala Ile Pro Ala Asp His Ile Val Leu Met Ala Gly Phe Thr Ala
            180                 185                 190

ggt aat gat aag ggc gag ctg gtg gtg ctg ggc cgc aac ggc tcc gac       624

Gly Asn Asp Lys Gly Glu Leu Val Val Leu Gly Arg Asn Gly Ser Asp
        195                 200                 205

tat tcc gcc gcg gtg ctg gcc gcc tgc ctg cgc gcc gac tgt tgc gag      672
Tyr Ser Ala Ala Val Leu Ala Ala Cys Leu Arg Ala Asp Cys Cys Glu
    210                 215                 220

atc tgg acc gac gtc gac ggc gtt tat acc tgc gat ccg cgc acc gtg      720
Ile Trp Thr Asp Val Asp Gly Val Tyr Thr Cys Asp Pro Arg Thr Val
225                 230                 235                 240

ccg gac gcc agg tta ctg aaa tcg atg tcg tac cag gaa gcg atg gag      768
Pro Asp Ala Arg Leu Leu Lys Ser Met Ser Tyr Gln Glu Ala Met Glu
                245                 250                 255

ctt tcc tat ttc ggc gcc aaa gtg tta cac ccc cgc acc atc act ccg      816
Leu Ser Tyr Phe Gly Ala Lys Val Leu His Pro Arg Thr Ile Thr Pro
            260                 265                 270

att gcc cag ttt caa atc cct tgc ctg att aaa aac acc tcc aac ccg      864
Ile Ala Gln Phe Gln Ile Pro Cys Leu Ile Lys Asn Thr Ser Asn Pro
        275                 280                 285

cag gcc ccc ggc acg ctg atc ggc aaa gac agc acc gat gcc gat atg      912
Gln Ala Pro Gly Thr Leu Ile Gly Lys Asp Ser Thr Asp Ala Asp Met
    290                 295                 300

ccg gtg aag ggc atc acc aac ctg aac aac atg gcg atg atc aac gtc      960
Pro Val Lys Gly Ile Thr Asn Leu Asn Asn Met Ala Met Ile Asn Val
305                 310                 315                 320

tcc ggc ccg ggc atg aaa ggc atg gtc ggc atg gcc gca cgg gtc ttc     1008
Ser Gly Pro Gly Met Lys Gly Met Val Gly Met Ala Ala Arg Val Phe
                325                 330                 335

gcc gtg atg tcg cgc gcc ggc att tcg gtg gtg ctg atc acc caa tcc     1056
Ala Val Met Ser Arg Ala Gly Ile Ser Val Val Leu Ile Thr Gln Ser
            340                 345                 350

tct tcc gaa tac agc atc agc ttc tgc gtg ccg cag ggt gaa ctg cag     1104
Ser Ser Glu Tyr Ser Ile Ser Phe Cys Val Pro Gln Gly Glu Leu Gln
        355                 360                 365

cgc gcc cgt cgc gcg ctg gaa gag gag ttc tat ctg gag ctg aaa gac     1152
Arg Ala Arg Arg Ala Leu Glu Glu Glu Phe Tyr Leu Glu Leu Lys Asp
    370                 375                 380

ggc gtg ctg gat ccg ctg gac gtg atg gaa cgc ctg gcg atc atc tcg     1200
Gly Val Leu Asp Pro Leu Asp Val Met Glu Arg Leu Ala Ile Ile Ser
385                 390                 395                 400

gtg gtt ggc gac ggc atg cgc acc ctg cgc ggc att tcc gca cgc ttc     1248
Val Val Gly Asp Gly Met Arg Thr Leu Arg Gly Ile Ser Ala Arg Phe
                405                 410                 415

ttc tcc gcg ctg gcg cgc gcc aac atc aat atc gtt gcc atc gcc caa     1296
Phe Ser Ala Leu Ala Arg Ala Asn Ile Asn Ile Val Ala Ile Ala Gln
            420                 425                 430

ggg tct tcc gaa cgt tcc att tcg gtg gtg gtc agc aat gat tcc gcc     1344
Gly Ser Ser Glu Arg Ser Ile Ser Val Val Val Ser Asn Asp Ser Ala
        435                 440                 445

acc acc ggc gtg cgc gtc agc cac cag atg ctg ttc aac acc gat cag     1392
Thr Thr Gly Val Arg Val Ser His Gln Met Leu Phe Asn Thr Asp Gln
    450                 455                 460

gtg atc gaa gtg ttc gtc atc ggc gtc ggc ggc gtc ggc ggg gcg ctg     1440
Val Ile Glu Val Phe Val Ile Gly Val Gly Gly Val Gly Gly Ala Leu

465                 470                 475                 480

atc gag cag atc tat cgc cag cag ccg tgg ctg aag caa aaa cat atc      1488
Ile Glu Gln Ile Tyr Arg Gln Gln Pro Trp Leu Lys Gln Lys His Ile
                485                 490                 495

gat ctg cgg gtg tgc ggc atc gcc aac tcg cgc gtg atg ctg acc aat      1536
Asp Leu Arg Val Cys Gly Ile Ala Asn Ser Arg Val Met Leu Thr Asn
            500                 505                 510

gtg cac ggc atc gcg ctg gac agc tgg cgc gac gcg ctg gcc ggc gcg      1584
Val His Gly Ile Ala Leu Asp Ser Trp Arg Asp Ala Leu Ala Gly Ala
        515                 520                 525

cag gag ccg ttc aat ctc ggc cgt ctg atc cgg ctg gtg aag gaa tat      1632
Gln Glu Pro Phe Asn Leu Gly Arg Leu Ile Arg Leu Val Lys Glu Tyr
    530                 535                 540

cac ctg ctg aac ccg gtg atc gtc gac tgt acc tcc agt cag gcg gtg      1680
His Leu Leu Asn Pro Val Ile Val Asp Cys Thr Ser Ser Gln Ala Val
545                 550                 555                 560

gcc gat cag tac gtg gac ttc ctg gcg gac ggc ttc cat gtg gtg acg      1728
Ala Asp Gln Tyr Val Asp Phe Leu Ala Asp Gly Phe His Val Val Thr
                565                 570                 575

ccg aac aaa aag gcc aac acc tcg tcg atg aac tat tat cag caa ctg      1776
Pro Asn Lys Lys Ala Asn Thr Ser Ser Met Asn Tyr Tyr Gln Gln Leu
            580                 585                 590

cgc gcg gcc gcc gcc ggt tcg cat cgc aag ttc ctg tac gac acc aac      1824
Arg Ala Ala Ala Ala Gly Ser His Arg Lys Phe Leu Tyr Asp Thr Asn
        595                 600                 605

gtc ggt gcc ggt ctg ccg gtg att gag aac ctg caa aac ctg ctg aac      1872
Val Gly Ala Gly Leu Pro Val Ile Glu Asn Leu Gln Asn Leu Leu Asn
    610                 615                 620

gcc ggt gat gaa ctg gta cgt ttc tcc ggt atc ctg tcc ggc tcg ctg      1920
Ala Gly Asp Glu Leu Val Arg Phe Ser Gly Ile Leu Ser Gly Ser Leu
625                 630                 635                 640

tcc ttt atc ttc ggc aag ctg gac gaa ggg ctg tcg ctg tcg gcg gcg      1968
Ser Phe Ile Phe Gly Lys Leu Asp Glu Gly Leu Ser Leu Ser Ala Ala
                645                 650                 655

acc ctg cag gcc cgg gcc aac ggc tac acc gag ccg gat ccg cgc gac      2016
Thr Leu Gln Ala Arg Ala Asn Gly Tyr Thr Glu Pro Asp Pro Arg Asp
            660                 665                 670

gat ctg tcc ggg atg gat gtg gcg cgt aag ctg ctg atc ctg gcg cgc      2064
Asp Leu Ser Gly Met Asp Val Ala Arg Lys Leu Leu Ile Leu Ala Arg
        675                 680                 685

gag gcc ggt tac aaa ctg gaa ctg agc gat atc gag gtc gag ccg gtg      2112
Glu Ala Gly Tyr Lys Leu Glu Leu Ser Asp Ile Glu Val Glu Pro Val
    690                 695                 700

ctg ccg ccg tcc ttc gac gcg tcg ggc gac gtg gac acc ttc ctg gcg      2160
Leu Pro Pro Ser Phe Asp Ala Ser Gly Asp Val Asp Thr Phe Leu Ala
705                 710                 715                 720

cgg ttg ccg gag ctg gat aaa gag ttc gcg cgc aac gtg gct aac gcc      2208
Arg Leu Pro Glu Leu Asp Lys Glu Phe Ala Arg Asn Val Ala Asn Ala
                725                 730                 735

gcc gag caa ggc aag gtg ctg cgc tat gtc ggc ctg atc gac gaa ggg      2256
Ala Glu Gln Gly Lys Val Leu Arg Tyr Val Gly Leu Ile Asp Glu Gly
            740                 745                 750

cgc tgc aag gtg cgc att gag gcg gtg gac ggc aac gat ccg ttg tat     2304
Arg Cys Lys Val Arg Ile Glu Ala Val Asp Gly Asn Asp Pro Leu Tyr
        755                 760                 765

aaa gtg aag aac ggc gag aac gcg ctg gcc ttc tac agc cgc tac tat     2352
Lys Val Lys Asn Gly Glu Asn Ala Leu Ala Phe Tyr Ser Arg Tyr Tyr
    770                 775                 780

cag ccg ttg ccg ctg gtg ctg cgc ggc tac ggc gcc ggt aac gat gtg     2400
Gln Pro Leu Pro Leu Val Leu Arg Gly Tyr Gly Ala Gly Asn Asp Val
785                 790                 795                 800

act gca gcg ggc gta ttc gcc gat ctg ctg cgc aca ctg tca tgg aag     2448
Thr Ala Ala Gly Val Phe Ala Asp Leu Leu Arg Thr Leu Ser Trp Lys
                805                 810                 815

ttg gga gtt taa                                                     2460
Leu Gly Val

<210>  10
<211>  819
<212>  PRT
<213>  Serratia marcescens

<400>  10

Met Arg Val Leu Lys Phe Gly Gly Thr Ser Val Ala Asn Ala Glu Arg
1               5                   10                  15

Phe Leu Arg Val Ala Asp Ile Met Glu Ser Asn Ala Arg Gln Gly Gln
            20                  25                  30

Val Ala Thr Val Leu Ser Ala Pro Ala Lys Ile Thr Asn His Leu Val
        35                  40                  45

Ala Met Ile Asp Lys Thr Val Ala Gly Gln Asp Ile Leu Pro Asn Met
    50                  55                  60

Ser Asp Ala Glu Arg Ile Phe Ala Asp Leu Leu Ser Gly Leu Ala Gln
65                  70                  75                  80

Ala Leu Pro Gly Phe Glu Tyr Asp Arg Leu Lys Gly Val Val Asp Gln
                85                  90                  95

Glu Phe Ala Gln Leu Lys Gln Val Leu His Gly Val Ser Leu Leu Gly
            100                 105                 110

Gln Cys Pro Asp Ser Val Asn Ala Ala Ile Ile Cys Arg Gly Glu Lys
        115                 120                 125

Leu Ser Ile Ala Ile Met Glu Gly Val Phe Arg Ala Lys Gly Tyr Pro
    130                 135                 140

Val Thr Val Ile Asn Pro Val Glu Lys Leu Leu Ala Gln Gly His Tyr
145                 150                 155                 160

Leu Glu Ser Thr Val Asp Ile Ala Glu Ser Thr Leu Arg Ile Ala Ala
                165                 170                 175

Ala Ala Ile Pro Ala Asp His Ile Val Leu Met Ala Gly Phe Thr Ala
            180                 185                 190

Gly Asn Asp Lys Gly Glu Leu Val Val Leu Gly Arg Asn Gly Ser Asp
        195                 200                 205

Tyr Ser Ala Ala Val Leu Ala Ala Cys Leu Arg Ala Asp Cys Cys Glu
    210                 215                 220

Ile Trp Thr Asp Val Asp Gly Val Tyr Thr Cys Asp Pro Arg Thr Val
225                 230                 235                 240

Pro Asp Ala Arg Leu Leu Lys Ser Met Ser Tyr Gln Glu Ala Met Glu
                245                 250                 255

Leu Ser Tyr Phe Gly Ala Lys Val Leu His Pro Arg Thr Ile Thr Pro
            260                 265                 270

Ile Ala Gln Phe Gln Ile Pro Cys Leu Ile Lys Asn Thr Ser Asn Pro
        275                 280                 285

Gln Ala Pro Gly Thr Leu Ile Gly Lys Asp Ser Thr Asp Ala Asp Met
    290                 295                 300

Pro Val Lys Gly Ile Thr Asn Leu Asn Asn Met Ala Met Ile Asn Val
305                 310                 315                 320

Ser Gly Pro Gly Met Lys Gly Met Val Gly Met Ala Ala Arg Val Phe
                325                 330                 335

Ala Val Met Ser Arg Ala Gly Ile Ser Val Val Leu Ile Thr Gln Ser
            340                 345                 350

Ser Ser Glu Tyr Ser Ile Ser Phe Cys Val Pro Gln Gly Glu Leu Gln
        355                 360                 365

Arg Ala Arg Arg Ala Leu Glu Glu Glu Phe Tyr Leu Glu Leu Lys Asp
    370                 375                 380

Gly Val Leu Asp Pro Leu Asp Val Met Glu Arg Leu Ala Ile Ile Ser
385                 390                 395                 400

Val Val Gly Asp Gly Met Arg Thr Leu Arg Gly Ile Ser Ala Arg Phe
                405                 410                 415

Phe Ser Ala Leu Ala Arg Ala Asn Ile Asn Ile Val Ala Ile Ala Gln
            420                 425                 430

Gly Ser Ser Glu Arg Ser Ile Ser Val Val Val Ser Asn Asp Ser Ala

435                 440                 445

Thr Thr Gly Val Arg Val Ser His Gln Met Leu Phe Asn Thr Asp Gln
    450                 455                 460

Val Ile Glu Val Phe Val Ile Gly Val Gly Gly Val Gly Gly Ala Leu
465                 470                 475                 480

Ile Glu Gln Ile Tyr Arg Gln Gln Pro Trp Leu Lys Gln Lys His Ile
                485                 490                 495

Asp Leu Arg Val Cys Gly Ile Ala Asn Ser Arg Val Met Leu Thr Asn
            500                 505                 510

Val His Gly Ile Ala Leu Asp Ser Trp Arg Asp Ala Leu Ala Gly Ala
        515                 520                 525

Gln Glu Pro Phe Asn Leu Gly Arg Leu Ile Arg Leu Val Lys Glu Tyr
    530                 535                 540

His Leu Leu Asn Pro Val Ile Val Asp Cys Thr Ser Ser Gln Ala Val
545                 550                 555                 560

Ala Asp Gln Tyr Val Asp Phe Leu Ala Asp Gly Phe His Val Val Thr
                565                 570                 575

Pro Asn Lys Lys Ala Asn Thr Ser Ser Met Asn Tyr Tyr Gln Gln Leu
            580                 585                 590

Arg Ala Ala Ala Ala Gly Ser His Arg Lys Phe Leu Tyr Asp Thr Asn
        595                 600                 605

Val Gly Ala Gly Leu Pro Val Ile Glu Asn Leu Gln Asn Leu Leu Asn
    610                 615                 620

Ala Gly Asp Glu Leu Val Arg Phe Ser Gly Ile Leu Ser Gly Ser Leu
625                 630                 635                 640

Ser Phe Ile Phe Gly Lys Leu Asp Glu Gly Leu Ser Leu Ser Ala Ala
                645                 650                 655

Thr Leu Gln Ala Arg Ala Asn Gly Tyr Thr Glu Pro Asp Pro Arg Asp
            660                 665                 670

Asp Leu Ser Gly Met Asp Val Ala Arg Lys Leu Leu Ile Leu Ala Arg
        675                 680                 685

Glu Ala Gly Tyr Lys Leu Glu Leu Ser Asp Ile Glu Val Glu Pro Val
    690                 695                 700

Leu Pro Pro Ser Phe Asp Ala Ser Gly Asp Val Asp Thr Phe Leu Ala
705                 710                 715                 720

Arg Leu Pro Glu Leu Asp Lys Glu Phe Ala Arg Asn Val Ala Asn Ala
                725                 730                 735

Ala Glu Gln Gly Lys Val Leu Arg Tyr Val Gly Leu Ile Asp Glu Gly
            740                 745                 750

Arg Cys Lys Val Arg Ile Glu Ala Val Asp Gly Asn Asp Pro Leu Tyr
        755                 760                 765

Lys Val Lys Asn Gly Glu Asn Ala Leu Ala Phe Tyr Ser Arg Tyr Tyr
    770                 775                 780

Gln Pro Leu Pro Leu Val Leu Arg Gly Tyr Gly Ala Gly Asn Asp Val
785                 790                 795                 800

Thr Ala Ala Gly Val Phe Ala Asp Leu Leu Arg Thr Leu Ser Trp Lys
                805                 810                 815

Leu Gly Val

<210>  11
<211>  1650
<212>  DNA
<213>  Escherichia coli

<220>
<221>  CDS
<222>  (1)..(1650)
<223>  yjcG-Kodierregion

<400>  11
atg aaa aga gtt ctg acg gcg ctt gcc gcc aca ctc cct ttc gca gct       48
Met Lys Arg Val Leu Thr Ala Leu Ala Ala Thr Leu Pro Phe Ala Ala
1               5                   10                  15

aac gcc gcg gat gct att agc ggg gcc gta gag cgc cag cca acg aac       96
Asn Ala Ala Asp Ala Ile Ser Gly Ala Val Glu Arg Gln Pro Thr Asn
            20                  25                  30

tgg cag gcg att att atg ttc ctg att ttc gtc gtg ttt acg ctc ggc      144
Trp Gln Ala Ile Ile Met Phe Leu Ile Phe Val Val Phe Thr Leu Gly
        35                  40                  45

att acc tac tgg gca tca aaa cgc gta cgt tct cgt agc gac tac tac      192
Ile Thr Tyr Trp Ala Ser Lys Arg Val Arg Ser Arg Ser Asp Tyr Tyr
    50                  55                  60

acc gca ggc ggc aat atc act ggc ttc cag aac ggg ctg gcg att gcc      240
Thr Ala Gly Gly Asn Ile Thr Gly Phe Gln Asn Gly Leu Ala Ile Ala
65                  70                  75                  80

ggg gac tat atg tcc gcc gcc tca ttc ttg ggg atc tcc gcg ctg gtg      288
Gly Asp Tyr Met Ser Ala Ala Ser Phe Leu Gly Ile Ser Ala Leu Val
                85                  90                  95

ttt acc tcc ggc tat gac ggc tta att tac tcg ctg ggc ttc ctg gtg      336
Phe Thr Ser Gly Tyr Asp Gly Leu Ile Tyr Ser Leu Gly Phe Leu Val

            100                 105                 110

ggc tgg ccg atc att ttg ttc ctg att gcc gaa cgt ctg cgt aac ctg 384
Gly Trp Pro Ile Ile Leu Phe Leu Ile Ala Glu Arg Leu Arg Asn Leu
        115                 120                 125

ggg cgc tac acc ttt gcc gat gtg gcc tct tac cgt ctg aaa caa ggg 432
Gly Arg Tyr Thr Phe Ala Asp Val Ala Ser Tyr Arg Leu Lys Gln Gly
    130                 135                 140

ccg att cgt att ctt tcg gcc tgt ggt tct ctg gtg gtg gtg gcg ctt 480
Pro Ile Arg Ile Leu Ser Ala Cys Gly Ser Leu Val Val Val Ala Leu
145                 150                 155                 160

tac ctt atc gcc cag atg gtg ggc gca ggt aaa ctg atc gag ctg ctg 528
Tyr Leu Ile Ala Gln Met Val Gly Ala Gly Lys Leu Ile Glu Leu Leu
                165                 170                 175

ttt ggc ctt aac tat cac att gcg gtg gtg ctg gtc ggc gtg ctg atg 576
Phe Gly Leu Asn Tyr His Ile Ala Val Val Leu Val Gly Val Leu Met
            180                 185                 190

atg atg tac gtc ctg ttc ggc ggc atg ctg gcg acc acc tgg gtg caa 624
Met Met Tyr Val Leu Phe Gly Gly Met Leu Ala Thr Thr Trp Val Gln
        195                 200                 205

att atc aaa gcc gtg ctg ttg ctg ttc ggt gcc agc ttt atg gcc ttt 672
Ile Ile Lys Ala Val Leu Leu Leu Phe Gly Ala Ser Phe Met Ala Phe
    210                 215                 220

atg gtg atg aaa cac gtc ggc ttt agc ttc aac aat ctg ttc agt gaa 720
Met Val Met Lys His Val Gly Phe Ser Phe Asn Asn Leu Phe Ser Glu
225                 230                 235                 240

gcg atg gcg gta cac ccg aaa ggt gtc gac atc atg aag ccg ggc ggg 768
Ala Met Ala Val His Pro Lys Gly Val Asp Ile Met Lys Pro Gly Gly
                245                 250                 255

ctg gtg aaa gat ccg atc tcc gcg ctc tct ctg ggt ctg gga ctg atg 816
Leu Val Lys Asp Pro Ile Ser Ala Leu Ser Leu Gly Leu Gly Leu Met
            260                 265                 270

ttt ggt acg gcg ggc ttg ccg cac att ctg atg cgc ttc ttt aca gtc 864
Phe Gly Thr Ala Gly Leu Pro His Ile Leu Met Arg Phe Phe Thr Val
        275                 280                 285

agc gat gcc cgc gaa gca cgt aag agc gtg ttc tac gcc acc ggg ttt 912
Ser Asp Ala Arg Glu Ala Arg Lys Ser Val Phe Tyr Ala Thr Gly Phe
    290                 295                 300

atg ggc tac ttc tat att ctg acc ttt att atc ggc ttc ggc gcg atc 960
Met Gly Tyr Phe Tyr Ile Leu Thr Phe Ile Ile Gly Phe Gly Ala Ile
305                 310                 315                 320

atg ctg gtt ggt gcg aat ccg gaa tat aaa gac gcg gcg ggc cat ctg 1008
Met Leu Val Gly Ala Asn Pro Glu Tyr Lys Asp Ala Ala Gly His Leu
                325                 330                 335

att ggt ggt aac aac atg gcg gcc gtt cac ctg gcg aat gca gtg ggc 1056
Ile Gly Gly Asn Asn Met Ala Ala Val His Leu Ala Asn Ala Val Gly
            340                 345                 350

ggc aac ctg ttc ctc ggt ttt att tca gcg gtt gct ttc gcc act atc 1104
Gly Asn Leu Phe Leu Gly Phe Ile Ser Ala Val Ala Phe Ala Thr Ile
        355                 360                 365

ctc gcg gtg gtt gcg ggt ctg acg ctg gcg ggc gca tcc gcg gtt tcg 1152
Leu Ala Val Val Ala Gly Leu Thr Leu Ala Gly Ala Ser Ala Val Ser
    370                 375                 380

cat gac ttg tac gct aac gtc ttc aaa aaa ggc gcg acc gaa cgt gaa 1200
His Asp Leu Tyr Ala Asn Val Phe Lys Lys Gly Ala Thr Glu Arg Glu
385                 390                 395                 400

gag ctg cgg gta tca aaa atc acc gta ctg atc ctc ggc gtg att gcg 1248
Glu Leu Arg Val Ser Lys Ile Thr Val Leu Ile Leu Gly Val Ile Ala
                405                 410                 415

att atc ctc ggc gtg ctg ttt gag aat cag aac atc gcc ttt atg gtg 1296
Ile Ile Leu Gly Val Leu Phe Glu Asn Gln Asn Ile Ala Phe Met Val
            420                 425                 430

ggg ctg gcg ttt gcc atc gcg gcg agc tgt aac ttc ccg atc att ctg 1344
Gly Leu Ala Phe Ala Ile Ala Ala Ser Cys Asn Phe Pro Ile Ile Leu
        435                 440                 445

ctt tct atg tac tgg tcg aaa ctg acc acg cgt ggc gcg atg atg ggt 1392
Leu Ser Met Tyr Trp Ser Lys Leu Thr Thr Arg Gly Ala Met Met Gly
    450                 455                 460

ggc tgg ctg ggg ctg att acc gca gta gta ctg atg atc ctc ggc ccg 1440
Gly Trp Leu Gly Leu Ile Thr Ala Val Val Leu Met Ile Leu Gly Pro
465                 470                 475                 480

acg att tgg gta cag atc ctt ggt cac gaa aaa gcc atc ttc ccg tat 1488
Thr Ile Trp Val Gln Ile Leu Gly His Glu Lys Ala Ile Phe Pro Tyr
                485                 490                 495

gaa tac ccg gcg ctg ttc tct atc acc gtg gca ttc ctc ggc atc tgg 1536
Glu Tyr Pro Ala Leu Phe Ser Ile Thr Val Ala Phe Leu Gly Ile Trp
            500                 505                 510

ttc ttc tcg gca acc gat aac tca gcg gaa ggc gcg cgt gag cgt gaa 1584
Phe Phe Ser Ala Thr Asp Asn Ser Ala Glu Gly Ala Arg Glu Arg Glu
        515                 520                 525

ctg ttc cgc gcg cag ttt atc cgc tcc cag acc ggc ttt ggc gtt gag 1632
Leu Phe Arg Ala Gln Phe Ile Arg Ser Gln Thr Gly Phe Gly Val Glu
    530                 535                 540

caa ggc cgc gcg cat taa 1650
Gln Gly Arg Ala His
545

<210> 12
<211> 549
<212> PRT
<213> Escherichia coli

<400> 12

Met Lys Arg Val Leu Thr Ala Leu Ala Ala Thr Leu Pro Phe Ala Ala
1               5                   10                  15

Asn Ala Ala Asp Ala Ile Ser Gly Ala Val Glu Arg Gln Pro Thr Asn
            20                  25                  30

Trp Gln Ala Ile Ile Met Phe Leu Ile Phe Val Val Phe Thr Leu Gly
        35                  40                  45

Ile Thr Tyr Trp Ala Ser Lys Arg Val Arg Ser Arg Ser Asp Tyr Tyr
    50                  55                  60

Thr Ala Gly Gly Asn Ile Thr Gly Phe Gln Asn Gly Leu Ala Ile Ala
65                  70                  75                  80

Gly Asp Tyr Met Ser Ala Ala Ser Phe Leu Gly Ile Ser Ala Leu Val
                85                  90                  95

Phe Thr Ser Gly Tyr Asp Gly Leu Ile Tyr Ser Leu Gly Phe Leu Val
            100                 105                 110

Gly Trp Pro Ile Ile Leu Phe Leu Ile Ala Glu Arg Leu Arg Asn Leu
        115                 120                 125

Gly Arg Tyr Thr Phe Ala Asp Val Ala Ser Tyr Arg Leu Lys Gln Gly
    130                 135                 140

Pro Ile Arg Ile Leu Ser Ala Cys Gly Ser Leu Val Val Val Ala Leu
145                 150                 155                 160

Tyr Leu Ile Ala Gln Met Val Gly Ala Gly Lys Leu Ile Glu Leu Leu
                165                 170                 175

Phe Gly Leu Asn Tyr His Ile Ala Val Val Leu Val Gly Val Leu Met
            180                 185                 190

Met Met Tyr Val Leu Phe Gly Gly Met Leu Ala Thr Thr Trp Val Gln
        195                 200                 205

Ile Ile Lys Ala Val Leu Leu Leu Phe Gly Ala Ser Phe Met Ala Phe
    210                 215                 220

Met Val Met Lys His Val Gly Phe Ser Phe Asn Asn Leu Phe Ser Glu
225                 230                 235                 240

Ala Met Ala Val His Pro Lys Gly Val Asp Ile Met Lys Pro Gly Gly
                245                 250                 255

Leu Val Lys Asp Pro Ile Ser Ala Leu Ser Leu Gly Leu Gly Leu Met
            260                 265                 270

Phe Gly Thr Ala Gly Leu Pro His Ile Leu Met Arg Phe Phe Thr Val
        275                 280                 285

Ser Asp Ala Arg Glu Ala Arg Lys Ser Val Phe Tyr Ala Thr Gly Phe
    290                 295                 300

Met Gly Tyr Phe Tyr Ile Leu Thr Phe Ile Ile Gly Phe Gly Ala Ile
305                 310                 315                 320

Met Leu Val Gly Ala Asn Pro Glu Tyr Lys Asp Ala Ala Gly His Leu
                325                 330                 335

Ile Gly Gly Asn Asn Met Ala Ala Val His Leu Ala Asn Ala Val Gly
            340                 345                 350

Gly Asn Leu Phe Leu Gly Phe Ile Ser Ala Val Ala Phe Ala Thr Ile
        355                 360                 365

Leu Ala Val Val Ala Gly Leu Thr Leu Ala Gly Ala Ser Ala Val Ser
    370                 375                 380

His Asp Leu Tyr Ala Asn Val Phe Lys Lys Gly Ala Thr Glu Arg Glu
385                 390                 395                 400

Glu Leu Arg Val Ser Lys Ile Thr Val Leu Ile Leu Gly Val Ile Ala
                405                 410                 415

Ile Ile Leu Gly Val Leu Phe Glu Asn Gln Asn Ile Ala Phe Met Val
            420                 425                 430

Gly Leu Ala Phe Ala Ile Ala Ala Ser Cys Asn Phe Pro Ile Ile Leu
        435                 440                 445

Leu Ser Met Tyr Trp Ser Lys Leu Thr Thr Arg Gly Ala Met Met Gly
    450                 455                 460

Gly Trp Leu Gly Leu Ile Thr Ala Val Val Leu Met Ile Leu Gly Pro
465                 470                 475                 480

Thr Ile Trp Val Gln Ile Leu Gly His Glu Lys Ala Ile Phe Pro Tyr
                485                 490                 495

Glu Tyr Pro Ala Leu Phe Ser Ile Thr Val Ala Phe Leu Gly Ile Trp
            500                 505                 510

Phe Phe Ser Ala Thr Asp Asn Ser Ala Glu Gly Ala Arg Glu Arg Glu
        515                 520                 525

Leu Phe Arg Ala Gln Phe Ile Arg Ser Gln Thr Gly Phe Gly Val Glu
    530                 535                 540

Gln Gly Arg Ala His
545

<210>  13

<211>  1035
<212>  DNA
<213>  Escherichia coli


<220>
<221>  CDS
<222>  (1)..(1035)
<223>  yibD-Kodierregion

<400>  13
atg atg aac agc acc aat aaa ctt agt gtt att att ccg tta tat aat        48
Met Met Asn Ser Thr Asn Lys Leu Ser Val Ile Ile Pro Leu Tyr Asn
1               5                   10                  15

gcg ggc gat gat ttc cgc act tgt atg gaa tct tta att acg caa acc        96
Ala Gly Asp Asp Phe Arg Thr Cys Met Glu Ser Leu Ile Thr Gln Thr
            20                  25                  30

tgg act gct ctg gaa atc att att att aac gat ggt tca acg gat aat       144
Trp Thr Ala Leu Glu Ile Ile Ile Ile Asn Asp Gly Ser Thr Asp Asn
        35                  40                  45

tct gtt gaa ata gca aag tat tac gca gaa aac tat ccg cac gtt cgt       192
Ser Val Glu Ile Ala Lys Tyr Tyr Ala Glu Asn Tyr Pro His Val Arg
    50                  55                  60

ttg ttg cat cag gcg aat gct ggc gca tcg gtg gcg cgt aat cgt ggg       240
Leu Leu His Gln Ala Asn Ala Gly Ala Ser Val Ala Arg Asn Arg Gly
65                  70                  75                  80

att gaa gtg gca acg ggc aaa tat gtc gct ttt gtc gat gct gac gat       288
Ile Glu Val Ala Thr Gly Lys Tyr Val Ala Phe Val Asp Ala Asp Asp
                85                  90                  95

gaa gtc tat ccc acc atg tac gaa acg ctg atg acc atg gcg tta gag       336
Glu Val Tyr Pro Thr Met Tyr Glu Thr Leu Met Thr Met Ala Leu Glu
            100                 105                 110

gac gac ctc gac gtg gcg cag tgc aac gct gac tgg tgt ttt cgt gaa       384
Asp Asp Leu Asp Val Ala Gln Cys Asn Ala Asp Trp Cys Phe Arg Glu
        115                 120                 125

acg gga gaa acc tgg caa tcc atc ccc acc gat cgc ctt cgc tca acc       432
Thr Gly Glu Thr Trp Gln Ser Ile Pro Thr Asp Arg Leu Arg Ser Thr
    130                 135                 140

ggc gta tta acc ggc ccg gac tgg ctg cgg atg ggg ctt tct tcg cgc       480
Gly Val Leu Thr Gly Pro Asp Trp Leu Arg Met Gly Leu Ser Ser Arg
145                 150                 155                 160

cgt tgg act cac gtt gtc tgg atg ggg gtt tat cgc cgt gat gtt att       528
Arg Trp Thr His Val Val Trp Met Gly Val Tyr Arg Arg Asp Val Ile
                165                 170                 175

gtt aaa aat aac att aaa ttt att gcc gga tta cat cat cag gat att       576
Val Lys Asn Asn Ile Lys Phe Ile Ala Gly Leu His His Gln Asp Ile
            180                 185                 190

gtc tgg aca aca gaa ttc atg ttt aac gcg ctg cgt gcg cga tat acc       624
Val Trp Thr Thr Glu Phe Met Phe Asn Ala Leu Arg Ala Arg Tyr Thr
        195                 200                 205

gag caa tca tta tat aaa tat tat ctg cat aat acg tca gtg agt cgg       672
Glu Gln Ser Leu Tyr Lys Tyr Tyr Leu His Asn Thr Ser Val Ser Arg
    210                 215                 220

ttg cat aga caa ggg aat aaa aac ctt aat tat caa cgt cac tat att 720
Leu His Arg Gln Gly Asn Lys Asn Leu Asn Tyr Gln Arg His Tyr Ile
225                 230                 235                 240

aag att acc cgc ctg ctg gag aaa tta aat cga aat tat gcc gac aaa 768
Lys Ile Thr Arg Leu Leu Glu Lys Leu Asn Arg Asn Tyr Ala Asp Lys
                245                 250                 255

att atg att tat ccg gaa ttt cat cag caa ata act tac gag gca ttg 816
Ile Met Ile Tyr Pro Glu Phe His Gln Gln Ile Thr Tyr Glu Ala Leu
            260                 265                 270

cgt gtt tgc cat gcg gtg cgc aaa gag ccg gat att ctt acc cgc caa 864
Arg Val Cys His Ala Val Arg Lys Glu Pro Asp Ile Leu Thr Arg Gln
        275                 280                 285

cgg atg att gcc gag ata ttt act tcc ggt atg tat aag cgc ctg att 912
Arg Met Ile Ala Glu Ile Phe Thr Ser Gly Met Tyr Lys Arg Leu Ile
    290                 295                 300

acc aat gtg cgc agc gtg aag gtc ggt tac cag gcg tta ctg tgg tct 960
Thr Asn Val Arg Ser Val Lys Val Gly Tyr Gln Ala Leu Leu Trp Ser
305                 310                 315                 320

ttc cgc tta tgg caa tgg cgc gac aaa acg cgg tcg cac cat cgc att 1008
Phe Arg Leu Trp Gln Trp Arg Asp Lys Thr Arg Ser His His Arg Ile
                325                 330                 335

acg cgt agc gcc ttt aat ttg cgc tag 1035
Thr Arg Ser Ala Phe Asn Leu Arg
            340

<210> 14
<211> 344
<212> PRT
<213> Escherichia coli

<400> 14

Met Met Asn Ser Thr Asn Lys Leu Ser Val Ile Ile Pro Leu Tyr Asn
1               5                   10                  15

Ala Gly Asp Asp Phe Arg Thr Cys Met Glu Ser Leu Ile Thr Gln Thr
            20                  25                  30

Trp Thr Ala Leu Glu Ile Ile Ile Ile Asn Asp Gly Ser Thr Asp Asn
        35                  40                  45

Ser Val Glu Ile Ala Lys Tyr Tyr Ala Glu Asn Tyr Pro His Val Arg
    50                  55                  60

Leu Leu His Gln Ala Asn Ala Gly Ala Ser Val Ala Arg Asn Arg Gly
65                  70                  75                  80

Ile Glu Val Ala Thr Gly Lys Tyr Val Ala Phe Val Asp Ala Asp Asp
                85                  90                  95

Glu Val Tyr Pro Thr Met Tyr Glu Thr Leu Met Thr Met Ala Leu Glu

            100                 105                 110

Asp Asp Leu Asp Val Ala Gln Cys Asn Ala Asp Trp Cys Phe Arg Glu
        115                 120                 125

Thr Gly Glu Thr Trp Gln Ser Ile Pro Thr Asp Arg Leu Arg Ser Thr
    130                 135                 140

Gly Val Leu Thr Gly Pro Asp Trp Leu Arg Met Gly Leu Ser Ser Arg
145                 150                 155                 160

Arg Trp Thr His Val Val Trp Met Gly Val Tyr Arg Arg Asp Val Ile
                165                 170                 175

Val Lys Asn Asn Ile Lys Phe Ile Ala Gly Leu His His Gln Asp Ile
            180                 185                 190

Val Trp Thr Thr Glu Phe Met Phe Asn Ala Leu Arg Ala Arg Tyr Thr
        195                 200                 205

Glu Gln Ser Leu Tyr Lys Tyr Tyr Leu His Asn Thr Ser Val Ser Arg
    210                 215                 220

Leu His Arg Gln Gly Asn Lys Asn Leu Asn Tyr Gln Arg His Tyr Ile
225                 230                 235                 240

Lys Ile Thr Arg Leu Leu Glu Lys Leu Asn Arg Asn Tyr Ala Asp Lys
                245                 250                 255

Ile Met Ile Tyr Pro Glu Phe His Gln Gln Ile Thr Tyr Glu Ala Leu
            260                 265                 270

Arg Val Cys His Ala Val Arg Lys Glu Pro Asp Ile Leu Thr Arg Gln
        275                 280                 285

Arg Met Ile Ala Glu Ile Phe Thr Ser Gly Met Tyr Lys Arg Leu Ile
    290                 295                 300

Thr Asn Val Arg Ser Val Lys Val Gly Tyr Gln Ala Leu Leu Trp Ser
305                 310                 315                 320

Phe Arg Leu Trp Gln Trp Arg Asp Lys Thr Arg Ser His His Arg Ile
                325                 330                 335

Thr Arg Ser Ala Phe Asn Leu Arg
            340

<210> 15
<211> 1332

<212>  DNA
<213>  Escherichia coli

<220>
<221>  CDS
<222>  (1)..(1332)
<223>  yaaU-Kodierregion

<400>  15
atg caa ccg tcc aga aac ttt gac gat ctc aaa ttc tcc tct att cac      48
Met Gln Pro Ser Arg Asn Phe Asp Asp Leu Lys Phe Ser Ser Ile His
1               5                   10                  15

cgc cgc att ttg ctg tgg gga agc ggt ggt ccg ttt ctg gat ggt tat      96
Arg Arg Ile Leu Leu Trp Gly Ser Gly Gly Pro Phe Leu Asp Gly Tyr
            20                  25                  30

gta ctg gta atg att ggc gtg gcg ctg gag caa ctg acg ccg gcg ctg      144
Val Leu Val Met Ile Gly Val Ala Leu Glu Gln Leu Thr Pro Ala Leu
        35                  40                  45

aaa ctg gac gct gac tgg att ggc ttg ctg ggc gcg gga acg ctc gcc      192
Lys Leu Asp Ala Asp Trp Ile Gly Leu Leu Gly Ala Gly Thr Leu Ala
    50                  55                  60

ggg ctg ttc gtt ggc aca tcg ctg ttt ggt tat att tcc gat aaa gtc      240
Gly Leu Phe Val Gly Thr Ser Leu Phe Gly Tyr Ile Ser Asp Lys Val
65                  70                  75                  80

gga cgg cgc aaa atg ttc ctc att gat atc atc gcc atc ggc gtg ata      288
Gly Arg Arg Lys Met Phe Leu Ile Asp Ile Ile Ala Ile Gly Val Ile
                85                  90                  95

tcg gtg gcg acg atg ttt gtt tca tcc ccc gtc gaa ctg ttg gtg atg      336
Ser Val Ala Thr Met Phe Val Ser Ser Pro Val Glu Leu Leu Val Met
            100                 105                 110

cgg gta ctt atc ggc att gtc atc ggt gca gat tat ccc atc gcc acc      384
Arg Val Leu Ile Gly Ile Val Ile Gly Ala Asp Tyr Pro Ile Ala Thr
        115                 120                 125

tca atg atc acc gag ttc tcc agt acc cgt cag cgg gcg ttt tcc atc      432
Ser Met Ile Thr Glu Phe Ser Ser Thr Arg Gln Arg Ala Phe Ser Ile
    130                 135                 140

agc ttt att gcc gcg atg tgg tat gtc ggc gcg acc tgt gcc gat ctg      480
Ser Phe Ile Ala Ala Met Trp Tyr Val Gly Ala Thr Cys Ala Asp Leu
145                 150                 155                 160

gtc ggc tac tgg ctt tat gat gtg gaa ggc ggc tgg cgc tgg atg ctg      528
Val Gly Tyr Trp Leu Tyr Asp Val Glu Gly Gly Trp Arg Trp Met Leu
                165                 170                 175

ggt agc gcg gcg atc ccc tgt ttg ttg att ttg att ggt cga ttc gaa      576
Gly Ser Ala Ala Ile Pro Cys Leu Leu Ile Leu Ile Gly Arg Phe Glu
            180                 185                 190

ctg cct gaa tct ccc cgc tgg tta tta cgc aaa ggg cga gta aaa gag      624
Leu Pro Glu Ser Pro Arg Trp Leu Leu Arg Lys Gly Arg Val Lys Glu
        195                 200                 205

tgc gaa gag atg atg atc aaa ctg ttt ggc gaa ccg gtg gct ttc gat      672
Cys Glu Glu Met Met Ile Lys Leu Phe Gly Glu Pro Val Ala Phe Asp
    210                 215                 220

gaa gag cag ccg cag caa acc cgt ttt cgc gat ctg ttt aat cgc cgc        720
Glu Glu Gln Pro Gln Gln Thr Arg Phe Arg Asp Leu Phe Asn Arg Arg
225                 230                 235                 240

cat ttt cct ttt gtt ctg ttt gtt gcc gcc atc tgg acc tgc cag gtg        768
His Phe Pro Phe Val Leu Phe Val Ala Ala Ile Trp Thr Cys Gln Val
                245                 250                 255

atc cca atg ttc gcc att tac acc ttt ggc ccg caa atc gtt ggt ttg        816
Ile Pro Met Phe Ala Ile Tyr Thr Phe Gly Pro Gln Ile Val Gly Leu
            260                 265                 270

ttg gga ttg ggg gtt ggc aaa aac gcg gca cta ggg aat gtg gtg att        864
Leu Gly Leu Gly Val Gly Lys Asn Ala Ala Leu Gly Asn Val Val Ile
        275                 280                 285

agc ctg ttc ttt atg ctc ggc tgt att ccg ccg atg ctg tgg tta aac        912
Ser Leu Phe Phe Met Leu Gly Cys Ile Pro Pro Met Leu Trp Leu Asn
    290                 295                 300

act gcc gga cgg cgt cca ttg ttg att ggc agc ttt gcc atg atg acg        960
Thr Ala Gly Arg Arg Pro Leu Leu Ile Gly Ser Phe Ala Met Met Thr
305                 310                 315                 320

ctg gcg ctg gcg gtt ttg ggg cta atc ccg gat atg ggg atc tgg ctg        1008
Leu Ala Leu Ala Val Leu Gly Leu Ile Pro Asp Met Gly Ile Trp Leu
                325                 330                 335

gta gtg atg gcc ttt gcg gtg tat gcc ttt ttc tct ggc ggg ccg ggt        1056
Val Val Met Ala Phe Ala Val Tyr Ala Phe Phe Ser Gly Gly Pro Gly
            340                 345                 350

aat ttg cag tgg ctc tat cct aat gaa ctc ttc ccg aca gat atc cgc        1104
Asn Leu Gln Trp Leu Tyr Pro Asn Glu Leu Phe Pro Thr Asp Ile Arg
        355                 360                 365

gcc tct gcc gtg ggc gtg att atg tcc tta agt cgt att ggc acc att        1152
Ala Ser Ala Val Gly Val Ile Met Ser Leu Ser Arg Ile Gly Thr Ile
    370                 375                 380

gtt tcg acc tgg gca cta ccg atc ttt atc aat aat tac ggt atc agt        1200
Val Ser Thr Trp Ala Leu Pro Ile Phe Ile Asn Asn Tyr Gly Ile Ser
385                 390                 395                 400

aac acg atg cta atg ggg gcg ggt atc tcg ctg ttt ggc ttg ttg att        1248
Asn Thr Met Leu Met Gly Ala Gly Ile Ser Leu Phe Gly Leu Leu Ile
                405                 410                 415

tcc gta gcg ttt gcc ccg gag act cga ggg atg tca ctg gcg cag acc        1296
Ser Val Ala Phe Ala Pro Glu Thr Arg Gly Met Ser Leu Ala Gln Thr
            420                 425                 430

agc aat atg acg atc cgc ggg cag aga atg ggg taa                        1332
Ser Asn Met Thr Ile Arg Gly Gln Arg Met Gly
        435                 440

<210>  16
<211>  443
<212>  PRT
<213>  Escherichia coli

<400>  16

Met Gln Pro Ser Arg Asn Phe Asp Asp Leu Lys Phe Ser Ser Ile His
1               5                   10                  15

Arg Arg Ile Leu Leu Trp Gly Ser Gly Gly Pro Phe Leu Asp Gly Tyr
            20                  25                  30

Val Leu Val Met Ile Gly Val Ala Leu Glu Gln Leu Thr Pro Ala Leu
        35                  40                  45

Lys Leu Asp Ala Asp Trp Ile Gly Leu Leu Gly Ala Gly Thr Leu Ala
    50                  55                  60

Gly Leu Phe Val Gly Thr Ser Leu Phe Gly Tyr Ile Ser Asp Lys Val
65                  70                  75                  80

Gly Arg Arg Lys Met Phe Leu Ile Asp Ile Ile Ala Ile Gly Val Ile
                85                  90                  95

Ser Val Ala Thr Met Phe Val Ser Ser Pro Val Glu Leu Leu Val Met
            100                 105                 110

Arg Val Leu Ile Gly Ile Val Ile Gly Ala Asp Tyr Pro Ile Ala Thr
        115                 120                 125

Ser Met Ile Thr Glu Phe Ser Ser Thr Arg Gln Arg Ala Phe Ser Ile
    130                 135                 140

Ser Phe Ile Ala Ala Met Trp Tyr Val Gly Ala Thr Cys Ala Asp Leu
145                 150                 155                 160

Val Gly Tyr Trp Leu Tyr Asp Val Glu Gly Gly Trp Arg Trp Met Leu
                165                 170                 175

Gly Ser Ala Ala Ile Pro Cys Leu Leu Ile Leu Ile Gly Arg Phe Glu
            180                 185                 190

Leu Pro Glu Ser Pro Arg Trp Leu Leu Arg Lys Gly Arg Val Lys Glu
        195                 200                 205

Cys Glu Glu Met Met Ile Lys Leu Phe Gly Glu Pro Val Ala Phe Asp
    210                 215                 220

Glu Glu Gln Pro Gln Gln Thr Arg Phe Arg Asp Leu Phe Asn Arg Arg
225                 230                 235                 240

His Phe Pro Phe Val Leu Phe Val Ala Ala Ile Trp Thr Cys Gln Val
                245                 250                 255

Ile Pro Met Phe Ala Ile Tyr Thr Phe Gly Pro Gln Ile Val Gly Leu
            260                 265                 270

Leu Gly Leu Gly Val Gly Lys Asn Ala Ala Leu Gly Asn Val Val Ile
        275                 280                 285

Ser Leu Phe Phe Met Leu Gly Cys Ile Pro Pro Met Leu Trp Leu Asn
    290                 295                 300

Thr Ala Gly Arg Arg Pro Leu Leu Ile Gly Ser Phe Ala Met Met Thr
305                 310                 315                 320

Leu Ala Leu Ala Val Leu Gly Leu Ile Pro Asp Met Gly Ile Trp Leu
                325                 330                 335

Val Val Met Ala Phe Ala Val Tyr Ala Phe Phe Ser Gly Gly Pro Gly
            340                 345                 350

Asn Leu Gln Trp Leu Tyr Pro Asn Glu Leu Phe Pro Thr Asp Ile Arg
        355                 360                 365

Ala Ser Ala Val Gly Val Ile Met Ser Leu Ser Arg Ile Gly Thr Ile
    370                 375                 380

Val Ser Thr Trp Ala Leu Pro Ile Phe Ile Asn Asn Tyr Gly Ile Ser
385                 390                 395                 400

Asn Thr Met Leu Met Gly Ala Gly Ile Ser Leu Phe Gly Leu Leu Ile
                405                 410                 415

Ser Val Ala Phe Ala Pro Glu Thr Arg Gly Met Ser Leu Ala Gln Thr
            420                 425                 430

Ser Asn Met Thr Ile Arg Gly Gln Arg Met Gly
        435                 440

## Beschreibung

Diese Erfindung betrifft ein Verfahren zur fermentativen Herstellung von L-Aminosäuren, insbesondere L-Threonin, L-Tryptophan, L-Lysin, L-Valin, L-Isoleucin und L-Homoserin, unter Verwendung von rekombinanten Mikroorganismen der Familie Enterobacteriaceae, in denen das glgS-Gen, welches für ein Protein kodiert, das als Regulator in die Glykogen-Biosynthese involviert ist, verstärkt, insbesondere überexprimiert wird, und diese Mikroorganismen.

### Stand der Technik

L-Aminosäuren, insbesondere L-Threonin, L-Tryptophan, L-Lysin, L-Valin, L-Isoleucin und L-Homoserin, finden in der Humanmedizin und in der pharmazeutischen Industrie, in der Lebensmittelindustrie und in der Tierernährung Anwendung.

Es ist bekannt, dass L-Aminosäuren durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie zum Beispiel die Auswahl des verwendeten Zuckers oder die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

In Wildtypstämmen verhindern strikte Regulationsmechanismen die über den Eigenbedarf hinausgehende Produktion von Stoffwechselprodukten wie Aminosäuren und deren Abgabe ins Medium. Die Konstruktion von aus Herstellersicht Aminosäure überproduzierenden Stämmen erfordert deshalb, diese Stoffwechselregulationen zu überwinden.

Zur Beseitigung der Kontrollmechanismen und Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren. Resistenz gegen das Tryptophan-Analogon 5-Methyl-DL-Tryptophan (5-MT) zeichnet zum Beispiel einen Stamm aus, der L-Tryptophan produziert.

Seit einigen Jahren werden ebenfalls Methoden der rekombinanten DNA-Technik zur gezielten Stammverbesserung L-Aminosäuren produzierender Stämme der Familie Enterobacteriaceae eingesetzt, indem man zum Beispiel einzelne Aminosäure-Biosynthesegene amplifiziert oder die Eigenschaften spezieller Gene verändert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2^{nd} edition, ASM Press, Washington, D.C., USA, (1996) zu finden. Eine Zusammenfassung zum Stoffwechsel und zur Produktion von L-Threonin ist veröffentlicht durch Debabov (Advances in Biochemical Engineering Vol.79, 113-136 (2003)).

Das Wachstumsphasen-regulierte und rpoS-abhängige glgS-Gen kodiert für ein Protein, das als Regulator in die Glykogen-Biosynthese involviert ist, die Überproduktion von GlgS stimuliert die Glykogen-Biosynthese, eine Transposoninsertion im glgS-Gen führt zu einer verringerten Glykogen-Biosynthese (Hengge-Aronis und Fischer; Molecular Microbiology 6(14):1877-1886 (1992); Hengge-Aronis et al., Journal of Bacteriology 175(1):259-265 (1993)). GlgS wirkt nicht auf die Expression des glgCAP-Operons, kodierend für zwei wesentliche Gene des Glykogen-Biosynthese-Weges und die Glykogen-Phosphorylase, einem Enzym des Glykogen-Abbauweges (Hengge-Aronis und Fischer; Molecular Microbiology 6(14):1877-1886 (1992)). Die glgS Expression wird negativ reguliert durch den Carbon Storage Regulator CsrA (Yang et al., Journal of Bacteriology 178(4):1012-1017 (1996)), die Struktur von GlgS wurde aufgeklärt durch Beglova et al. (European Journal of Biochemistry 246(2):301-310 (1997)) und Kozlov et al. (BMC Biology 2(1):10 (2004)) und deutet auf eine Funktion über eine Protein-Protein-Wechselwirkung hin.

Die Nukleotidsequenz der Wildform des glgS-Gens von Escherichia coli wurde von Blattner et al. (Science 277 (5331), 1453-1474 (1997)) bestimmt und ist in der Datenbank des National Center for Biotechnology Information (NCBI) der National Library of Medicin (Bethesda, MD, USA) unter den Zugangsnummern (accession number) U00096 (Region: komplementär(3189761-3189961)) sowie NC_000913.2 (Region: komplementär(3189761-3189961) allgemein verfügbar.

### Aufgabe der Erfindung

Die Erfinder haben sich die Aufgabe gestellt, neue Maßnahmen zur Herstellung erhöhter Mengen von L-Aminosäuren, insbesondere L-Threonin, L-Tryptophan, L-Lysin, L-Valin, L-Isoleucin und L-Homoserin, bereitzustellen.

### Beschreibung der Erfindung

Gegenstand der Erfindung sind rekombinante L-Aminosäure produzierende Mikroorganismen der Familie Enterobacteriaceae, die ein verstärktes oder überexprimiertes glgS-Gen, das für ein Protein kodiert, welches als Regulator in die Glykogen-Biosynthese involviert ist, oder für dessen Genprodukt kodierende Nukleotidsequenzen enthalten, und die vermehrt L-Aminosäuren, insbesondere L-Threonin, L-Tryptophan, L-Lysin, L-Valin, L-Isoleucin und L-Homoserin, produzieren und in der Zelle oder im Medium anreichern.

Als Ausgangspunkt für den Vergleich dienen jeweils die für das glgS-Gen nicht rekombinanten Mikroorganismen, die kein verstärktes glgS-Gen enthalten und an denen die erfindungsgemäße Verstärkung oder Überexpression nicht durchgeführt wurde.

Zu diesen Mikroorganismen gehören insbesondere Mikroorganismen der Familie Enterobacteriaceae, in denen ein Polynukleotid verstärkt wird, das für ein Polypeptid kodiert, dessen Aminosäuresequenz zu mindestens 80% oder mindestens 90%, insbesondere zu mindestens 95%, bevorzugt zu mindestens 98% oder mindestens 99%, besonders bevorzugt zu 99,6% und ganz besonders bevorzugt zu 100% identisch ist mit einer Aminosäuresequenz, ausgewählt aus der Gruppe SEQ ID No. 2, SEQ ID No. 4 und SEQ ID No. 6, bevorzugt SEQ ID No. 2, wobei das Polypeptid die Aktivität des Glykogen Synthese Proteins GlgS besitzt.

Die genannten Mikroorganismen enthalten isolierte Polynukleotide, ausgewählt aus der Gruppe:
a) Polynukleotid mit einer Nukleotidsequenz, ausgewählt aus SEQ ID No. 1, SEQ ID No. 3 oder SEQ ID No. 5 und den dazu komplementären Nukleotidsequenzen;
b) Polynukleotid mit einer Nukleotidsequenz, die der SEQ ID No. 1, SEQ ID No. 3 oder SEQ ID No. 5 im Rahmen der Degeneration des genetischen Codes entspricht;
c) Polynukleotidsequenz mit einer Sequenz, die mit der zur Sequenz SEQ ID No. 1, SEQ ID No. 3 oder SEQ ID No. 5 komplementären Sequenz unter stringenten Bedingungen hybridisiert, wobei die stringenten Bedingungen bevorzugt durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1, SEQ ID No. 3 oder SEQ ID No. 5, die funktionsneutrale Sinnmutanten enthält,
wobei die Polynukleotide für ein Protein kodieren, welches als Regulator in die Glykogen-Biosynthese involviert ist.

Gegenstand der Erfindung ist ebenso ein Verfahren zur fermentativen Herstellung von L-Aminosäuren, insbesondere L-Threonin, L-Tryptophan, L-Lysin, L-Valin, L-Isoleucin und L-Homoserin, unter Verwendung von rekombinanten Mikroorganismen der Familie Enterobacteriaceae, nicht von Wildtypstämmen, die insbesondere bereits vor der erfindungsgemäßen Verstärkung oder Überexpression L-Aminosäuren produzieren, und in denen mindestens das glgS-Gen oder für dessen Genprodukt kodierende Nukleotidsequenzen verstärkt wird bzw. werden.

Als vor der erfindungsgemäßen Verstärkung oder Überexpression L-Aminosäure produzierende Mikroorganismen zählen dabei nicht die Wildtypstämme und häufig benutzte Laborstämme wie unter anderen DH5α, DH5αmcr, W3110, MG1655, MC4100, Y1089, H560 und MM152.

Bevorzugt werden die beschriebenen Mikroorganismen eingesetzt.

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin gemeint. Besonders bevorzugt sind L-Threonin, L-Tryptophan, L-Lysin, L-Valin, L-Isoleucin und L-Homoserin.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert.

Die Konzentration des Proteins kann über 1- und 2-dimensionale Proteingelauftrennung und anschließende optische Identifizierung der Proteinkonzentration mit enstprechender Auswertesoftware im Gel bestimmt werden. Eine gebräuchliche Methode zur Präparation der Proteingele und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich, Angewandte Chemie 38: 2630-2647 (1999)) bestimmt werden.

Ein Gen oder Allel ist chemisch ein Polynukleotid. Ein anderer Begriff hierfür ist Nukleinsäure, insbesondere Desoxyribonukleinsäure.

Die Begriffe Polypeptid und Protein sind gegenseitig austauschbar.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfindungsgemäße Verstärkung oder Überexpression durchgeführt wird.

Gegenstand der Erfindung ist ein Verfahren zur Herstellung von L-Aminosäuren durch Fermentation der erfindungsgemäßen rekombinanten Mikroorganismen der Familie Enterobacteriaceae, dadurch gekennzeichnet, dass man
a) die die gewünschte L-Aminosäure produzierenden Mikroorganismen, in denen man das glgS-Gen oder für dessen Genprodukt kodierende Nukleotidsequenzen oder Allele verstärkt, insbesondere überexprimiert, in einem Medium kultiviert unter Bedingungen, bei denen die gewünschte L-Aminosäure im Medium oder in den Zellen angereichert wird, und,
b) die gewünschte L-Aminosäure isoliert, wobei gegebenenfalls weitere Bestandteile der Fermentationsbrühe und/oder die Biomasse in ihrer Gesamtheit oder Anteilen (≥ 0 bis 100 %) im isolierten Produkt verbleiben oder vollständig entfernt werden.

Die insbesondere rekombinanten Mikroorganismen mit einem verstärkten oder überexprimierten glgS-Gen, die ebenfalls Gegenstand der vorliegenden Erfindung sind, können L-Aminosäuren aus Glucose, Saccharose, Laktose, Fructose, Maltose, Melasse, gegebenenfalls Stärke, gegebenenfalls Cellulose oder aus Glycerin und Ethanol, gegebenenfalls auch aus Mischungen, herstellen. Es handelt sich um Vertreter der Familie Enterobacteriaceae, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Rekombinante Mikroorganismen werden im Allgemeinen durch Transformation, Transduktion oder Konjugation, oder einer Kombination dieser Methoden, mit einem Vektor erzeugt, der das gewünschte Gen, den gewünschten ORF, ein Allel dieses Gens oder ORFs oder Teile davon und/oder einen die Exprimierung des Gens oder ORFs verstärkenden Promotor enthält. Bei diesem Promotor kann es sich um den durch verstärkende Mutation aus der eigenen, upstream des Gens oder ORFs befindlichen regulatorischen Sequenz hervorgegangenen Promotor handeln, oder es wurde ein effizienter Promotor mit dem Gen oder ORF fusioniert.

Zur Herstellung der L-Aminosäure-anreichernden Stämme der Familie Enterbacteriaceae, welche ein verstärktes glgS-Gen enthalten, werden bevorzugt Stämme verwendet (Ausgangsstämme bzw. Elternstämme), die bereits die Fähigkeit besitzen, die gewünschte L-Aminosäure in der Zelle anzureichern und/oder in das sie umgebende Nährmedium auszuscheiden bzw. in der Fermentationsbrühe zu akkumulieren. Hierfür kann auch der Ausdruck "produzieren" verwendet werden. Insbesondere besitzen die für die Maßnahmen der Erfindung eingesetzten Stämme die Fähigkeit ≥ (mindestens) 0,25 g/l, ≥ 0,5 g/l, ≥ 1,0 g/l, ≥ 1,5 g/l, ≥ 2,0 g/l, ≥ 4 g/l oder ≥ 10 g/l an L-Aminosäure in ≤ (maximal) 120 Stunden, ≤ 96 Stunden, ≤ 48 Stunden, ≤ 36 Stunden, ≤ 24 Stunden oder ≤ 12 Stunden in der Zelle und/oder im Nährmedium bzw. der Fermentationsbrühe anzureichern bzw. zu akkumulieren. Hierbei kann es sich um Stämme handeln, die durch Mutagenese und Selektion, durch rekombinante DNA-Techniken oder durch eine Kombination beider Methoden hergestellt wurden.

Die genannten L-Aminosäuren-ausscheidenden Stämme produzieren eine oder mehrere, bevorzugt eine oder im Wesentlichen eine Aminosäure ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin, bevorzugt ausgewählt aus der Gruppe L-Threonin, L-Tryptophan, L-Lysin, L-Valin, L-Isoleucin und L-Homoserin. Der Begriff L-Aminosäure oder Aminosäure umfasst auch deren Salze.

Der Begriff " eine oder im Wesentlichen eine Aminosäure" berücksichtigt, dass zusätzlich zu der gewünschten L-Aminosäure eine oder mehrer weitere der genannten L-Aminosäuren (Nebenaminosäuren) produziert werden können. Der Anteil dieser Nebenaminosäuren beträgt ≥ 0 bis maximal 40%, bevorzugt ≥ 0 bis maximal 20%, besonders bevorzugt ≥ 0 bis maximal 10% und ganz besonders bevorzugt ≥ 0 bis maximal 5% bezogen auf die Menge der gewünschten L-Aminosäure.

Als zum Elternstamm geeignete, insbesondere L-Threonin produzierende bzw. ausscheidende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli TH 14.97 (WO 02/26993)
- Escherichia coli TH 21.97 (WO 02/26993)
- Escherichia coli BKIIM B-3996 (US-A-5,175,107)
- Escherichia coli BKIIM B-3996ΔtdhΔpckA/pVIC40 (WO 02/29080)
- Escherichia coli kat 13 (WO 98/04715)
- Escherichia coli Kat 69.9 (WO 02/26993)
- Escherichia coli KCCM-10132 (WO 00/09660)
- Escherichia coli KCCM-10168 (WO 01/14525)
- Escherichia coli KCCM-10133 (WO 00/09661)

Als zum Elternstamm geeignete L-Threonin produzierende bzw. ausscheidende Stämme der Gattung Serratia, insbesondere der Art Serratia marcescens sind beispielsweise zu nennen:
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (Applied Biochemistry and Biotechnology 37(3): 255-265 (1992))

L-Threonin produzierende oder ausscheidende Stämme aus der Familie der Enterobacteriaceae besitzen bevorzugt, unter anderen, ein oder mehrere der genetischen bzw. phänotypischen Merkmale ausgewählt aus der Gruppe:
Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Als Elternstamm geeignete, L-Tryptophan produzierende bzw. ausscheidende Stämme der Gattung Escherichia, insbesondere der Art Escherichia coli sind beispielsweise zu nennen:
- Escherichia coli JP4735/pMU3028 (US5,756,345)
- Escherichia coli JP6015/pMU91 (US5,756,345)
- Escherichia coli SV164(pGH5) (WO94/08031)
- E. coli AGX17(pGX44) (NRRL B-12263) (US4,371,614)
- E. coli AGX6(pGX50)aroP (NRRL B-12264) (US4,371,614)
- Escherichia coli AGX17/pGX50,pACKG4-pps (WO97/08333)
- Escherichia coli ATCC 31743 (CA1182409)
- E. coli C534/pD2310,pDM136 (ATCC 39795) (WO87/01130)
- Escherichia coli JB102/p5LRPS2 (US5,939,295).

L-Tryptophan produzierende oder ausscheidende Stämme aus der Familie der Enterobacteriaceae besitzen bevorzugt, unter anderen, ein oder mehrere der genetischen bzw. phänotypischen Merkmale ausgewählt aus der Gruppe:
Resistenz gegen 5-Methyl-DL-Tryptophan, Resistenz gegen 5-Fluoro-Tryptophan, Resistenz gegen 4-Methyl-DL-Tryptophan, Resistenz gegen 6-Methyl-DL-Tryptophan, Resistenz gegen 4-Fluoro-Tryptophan, Resistenz gegen 6-Fluoro-Tryptophan, Resistenz gegen Anthranilat, Resistenz gegen Tryptazan, Resistenz gegen Indol, Resistenz gegen Indol-Acrylsäure, Bedürftigkeit für Phenylalanin, Bedürftigkeit für Tyrosin, gegebenenfalls Fähigkeit zur Saccharose-Verwertung, Verstärkung des Tryptophan-Operons, bevorzugt der Anthranilat-Synthase bevorzugt der feed back resistenten Form, eine teilweise defekte Tryptophanyl-tRNA-Synthase, eine abgeschwächte Tryptophan-Aufnahme, eine defekte Tryptophanase, inaktivierte Repressorproteine, Verstärkung der Serin-Biosynthese, Verstärkung der Phophoenolpyruvat-Synthese, Verstärkung der D-Erythrose-4-Phosphat-Synthese, Verstärkung der 3-deoxy-D-arabino-heptulosonat-7-Phosphat(DHAP)-Synthese, Verstärkung der Chorismat-Biosynthese.

Als Elternstamm geeigneter, L-Homoserin ausscheidender bzw. produzierender Stamm der Gattung Escherichia, insbesondere der Art Escherichia coli ist beispielsweise zu nennen:

Escherichia coli NZ10rhtA23/pAL4 (US 6,960,455).

Als Elternstamm geeignete, L-Lysin ausscheidende bzw. produzierende Stämme der Gattung Escherichia, insbesondere der Art Escherichia coli sind beispielsweise zu nennen:
Escherichia coli pDA1/TOC21R (= CNCM 1-167) (FR-A-2511032)
Escherichia coli NRRL B-12199 (US4,346,170)
Escherichia coli NRRL B-12185 (US4,346,170).

Als Elternstamm geeignete, L-Isoleucin ausscheidende bzw. produzierende Stämme der Gattung Escherichia, insbesondere der Art Escherichia coli sind beispielsweise zu nennen:
Escherichia coli H-8670 (FERM BP-4051) (US5,460,958)
Escherichia coli H-8683 (FERM BP-4052) (US5,460,958)
Escherichia coli FERM BP-3628 (US5,362,637)
Escherichia coli FERM BP-3629 (US5,362,637)
Escherichia coli FERM BP-3630 (US5,362,637)
Escherichia coli H-9146 (FERM BP-5055) (US5,695,972)
Escherichia coli H-9156 (FERM BP-5056) (US5,695,972).

L-Isoleucin produzierende oder ausscheidende Stämme aus der Familie der Enterobacteriaceae besitzen bevorzugt, unter anderen, ein oder mehrere der genetischen bzw. phänotypischen Merkmale ausgewählt aus der Gruppe:
Resistenz gegen Isoleucin-Analoga wie Thiaisoleucin, Resistenz gegen Ethionin, Resistenz gegen Arginin-Hydroxamat, Resistenz gegen S-(2-Aminoethyl)-L-Cystein, Resistenz gegen D-Serin.

Als Elternstamm geeigneter, L-Valin ausscheidender bzw. produzierender Stamm der Gattung Escherichia, insbesondere der Art Escherichia coli ist beispielsweise zu nennen:
Escherichia coli AJ11502 (NRRL B-12288) (US-A-4391907).

Bei den der Erfindung zugrunde liegenden Arbeiten wurde gefunden, dass Mikroorganismen der Familie Enterobacteriaceae nach Überexpression des Gens glgS, oder dessen Allelen, vermehrt L-Aminosäuren, insbesondere L-Threonin, L-Tryptophan, L-Lysin, L-Valin, L-Isoleucin und L-Homoserin, produzieren und in der Zelle oder im Medium anreichern

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminopeptidase) die N-terminale Aminosäure Methionin abgespalten werden kann.

Aus den ebenfalls zur Familie Enterobacteriaceae gehörenden Shigella flexneri und Salmonella enterica sind die Nukleotidsequenzen für das glgS-Gen ebenso bekannt (Accession No.: AE005674 (REGION: komplementär(3181115-3181315) bzw. AE014613 (REGION: komplementär(3202310-3202513)).

Das glgS-Gen von Escherichia coli K12 wird unter anderem durch folgende Angaben beschrieben:

Das Genprodukt des glgS-Gens, ein 7892-Da Protein, ist als Regulator in die Glykogen-Biosynthese involviert. Die Expression des Gens wird kontrolliert von SigmaS (rpoS) und in der Stationärphase induziert (Hengge-Aronis und Fischer; Molecular Microbiology 6(14):1877-1886 (1992); Hengge-Aronis et al., Journal of Bacteriology 175(1):259-265 (1993); Beglova et al. (European Journal of Biochemistry 246(2):301-310 (1997)). Das kodierte Polypeptid GlgS besitzt eine Länge von 66 Aminosäuren.

| | |
|---|---|
| Accession No.: | NC_000913.2 (REGION |
| | Komplementär(3189761-3189961)) |
| Alternativer Genname: | b3049 |

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum glgS-Gen von Escherichia coli unter der SEQ ID No. 1 und die bekannten Sequenzen zum glgS-Gen von Shigella flexneri und Salmonella enterica unter der SEQ ID No. 3 bzw. SEQ ID No. 5 dargestellt. Die von diesen Leserahmen kodierten Proteine sind als SEQ ID No. 2, SEQ ID No. 4 und SEQ ID No. 6 dargestellt.

Die in den angegebenen Textstellen beschriebenen Gene oder offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben. Die Verwendung endogener Gene bzw. endogener offener Leserahmen wird bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Zu den Allelen des glgS-Gens, welche funktionsneutrale Sinnmutationen enthalten, zählen unter anderem solche, die zu höchstens 15, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5 in keinem Falle aber mehr als 10 Aminosäuren betreffen.

Zu den geeigneten Allelen gehören ferner solche die durch Hybridisierung, insbesondere unter stringenten Bedingungen unter Verwendung von SEQ ID No. 1, SEQ ID No. 3 oder SEQ ID No. 5 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der

Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1, SEQ ID No. 3 oder SEQ ID No. 5 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Zur Erzielung einer Verstärkung können beispielsweise die Expression der Gene oder offenen Leserahmen oder Allele oder die katalytischen Eigenschaften des Proteins erhöht werden. Gegebenenfalls können beide Maßnahmen kombiniert werden.

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Threonin-Produktion zu steigern, des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene, ORFs oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, lpp-Promotor oder P_{L}-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren, der nar-Promotor oder die Promotoren der Gene rrsG, rnpB, csrA, csrB, ompA, fusA, pepQ, rplX oder rpsG verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)). Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Der Austausch des Promotors gegen einen zum Beispiel in genomweit-vergleichenden Expressionsanalysen ermittelten Promotor mit gleichmäßig hoher Expression im Verlauf eines Fermentationsprozesses bewirkt eine gleichmäßige Verstärkung. Die Veränderung einer Wachstumsphasen-abhängigen Genexpression kann beispielsweise wie bei Walker et al. (Journal of Bacteriology 181: 1269-80 (1999)) beschrieben durch die Verwendung des Wachstumsphasen-abhängigen fis Promotors oder einer der dort beschriebenen Mutationen des fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden. Desweiteren kann der Austausch eines Start-Codons zu dem in Escherichia coli mit 77% am häufigsten vorkommenden Codon ATG die Translation erheblich verbessern, da das Codon AUG zwei- bis dreimal effektiver ist als zum Beispiel die Codons GUG und UUG (Khudyakov et al., FEBS Letters 232(2):369-71 (1988); Reddy et al., Proceedings of the National Academy of Sciences of the USA 82(17):5656-60 (1985)). Auch die Sequenzumgebung des Start-Codons kann optimiert werden, da zusammenwirkende Effekte zwischen dem Start-Codon und den flankierenden Bereichen beschrieben sind (Stenstrom et al., Gene 273(2):259-65 (2001); Hui et al., EMBO Journal 3(3):623-9 (1984)).

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolomé et al.; Gene 102: 75-78 (1991)), pTrc99A (Amann et al.; Gene 69: 301-315 (1988)), pBR322- oder pSC101-Derivate (Bolivar et al., Gene 2: 95-113 (1977); Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens das glgS-Gen, oder für dessen Genprodukt kodierende Nukleotidsequenzen oder Allele trägt.

Unter dem Begriff Transformation versteht man die Aufnahme einer isolierten Nukleinsäure durch einen Wirt (Mikroorganismus).

Zur Erzeugung von glgS-Allelen, die in den für das erfindungsgemäße Verfahren eingesetzten Mikroorganismen zum Einsatz kommen, können unter anderem im Stand der Technik beschriebene Methoden zur gerichteten Mutagenese verwendet werden.

Es können Verfahren der ortsgerichteten Mutagenese unter Verwendung mutagener Oligonukleotide (T. A. Brown: Gentechnologie für Einsteiger, Spektrum Akademischer Verlag, Heidelberg, 1993) oder der Polymerase-Kettenreaktion (PCR), wie sie im Handbuch von Gait: Oligonukleotide synthesis: A Practical Approach (IRL Press, Oxford, UK, 1984) oder von Newton und Graham (PCR, Spektrum Akademischer Verlag, Heidelberg, 1994) beschrieben sind, verwendet werden. Die erzeugten Mutationen können durch DNA-Sequenzierung beispielsweise nach der Methode von Sanger et al. (Proceedings of the National Academy of Science USA 74 (12): 5463-5467 (1977)) bestimmt und überprüft werden.

Zur Konstruktion von Punktmutationen im glgS-Gen kann zum Beispiel das Quick Change Site-Directed Mutagenesis Kit der Firma Stratagene (Amsterdam, Niederlande) verwendet werden. Bei Verwendung dieser Methoden wird das im Stand der Technik beschriebene glgS-Gen ausgehend von isolierter Gesamt-DNA eines Wildtypstammes mit Hilfe der Polymerase-Kettenreaktion (PCR) amplifiziert, in geeignete Plasmidvektoren kloniert, und die DNA anschließend dem Mutageneseverfahren unterworfen. Mittels "GeneSOEing" (Gene Splicing by Overlap Extension, Horton, Molecular Biotechnology 3: 93-98 (1995)) können die Punktmutationen schon per PCR erhalten werden.

Die erzeugten glgS-Allele können beispielsweise durch Transformation und das Verfahren des Gen- bzw. Allelaustausches in geeignete Stämme eingebaut werden.

Eine gebräuchliche Methode ist die von Hamilton et al. (Journal of Bacteriology 174, 4617 - 4622 (1989)) beschriebene Methode des Genaustauschs mit Hilfe eines konditional replizierenden pSC101-Derivates pMAK705 oder mit pK03 (Link et al., Journal of Bacteriology 179 : 6228-6237). Andere im Stand der Technik beschriebene Methoden wie beispielsweise die von Martinez-Morales et al. (Journal of Bacteriology 1999, 7143-7148 (1999)) oder die von Boyd et al. (Journal of Bacteriology 182, 842-847 (2000)) können gleichfalls benutzt werden.

Es ist ebenfalls möglich die erzeugten glgS-Allele durch Konjugation oder Transduktion in verschiedene Stämme zu überführen.

Die Mutationen von glgS können ebenfalls in Expressionsplasmiden durchgeführt werden, so dass die resultierenden Proteine überproduziert werden können.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Weiterhin kann es für die erhöhte Produktion von L-Aminosäuren, insbesondere L-Threonin, L-Tryptophan, L-Lysin, L-Valin, L-Isoleucin und L-Homoserin mit Stämmen der Familie Enterobacteriaceae vorteilhaft sein, zusätzlich zur Verstärkung des glgS-Gens, ein oder mehrere Enzym(e) des bekannten Threonin-Biosyntheseweges oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

Ebenso kann es für die Produktion von L-Trytophan mit Stämmen der Familie Enterobacteriaceae vorteilhaft sein, zusätzlich zur Verstärkung des glgS-Gens, ein oder mehrere Enzyme des bekannten Tryptophan-Biosyntheseweges oder Enzyme der Serin-Biosynthese oder Enzyme für die Produktion von 3-deoxy-D-arabino-heptulosonat-7-Phosphat (DHAP) und Chorismat zu verstärken. Die Verwendung endogener Gene wird im Allgemeinen bevorzugt.

So können beispielsweise für die Produktion von L-Threonin gleichzeitig eines oder mehrere der Gene, ausgewählt aus der Gruppe
mindestens ein Gen des für die Aspartatkinase, die Homoserin-Dehydrogenase, die Homoserinkinase und die Threoninsynthase kodierenden thrABC-Operons (US-A-4,278,765),
das für die Pyruvat-Carboxylase kodierende pyc-Gen von Corynebacterium glutamicum (WO 99/18228),
das für die Phosphoenolpyruvat-Synthase kodierende pps-Gen (Molecular and General Genetics 231(2): 332-336 (1992); WO 97/08333),
das für die Phosphoenolpyruvat-Carboxylase kodierende ppc-Gen (WO 02/064808),
die für die Untereinheiten der Transhydrogenase kodierenden Gene pntA und pntB (European Journal of Biochemistry 158: 647-653 (1986); WO 95/11985),
das für das Threoninresistenz vermittelnde Protein kodierende Gen rhtC (EP-A-1 013 765),
das für das Threonin-Export-Carrier-Protein kodierende thrE-Gen von Corynebacterium glutamicum (WO 01/92545),
das für die Glutamat-Dehydrogenase kodierende gdhA-Gen (Nucleic Acids Research 11: 5257-5266 (1983); Gene 23: 199-209 (1983); DE19907347),
das für die Phosphohistidin-Protein-Hexose-Phosphotransferase des Phosphotransferase-Systems PTS kodierende ptsH-Gen des ptsHIcrr-Operons (WO 03/004674),
das für das Enzym I des Phosphotransferase-Systems PTS kodierende ptsI-Gen des ptsHIcrr-Operons (WO 03/004674),
das für die Glucose-spezifische IIA Komponente des Phosphotransferase-Systems PTS kodierende crr-Gen des ptsHIcrr-Operons (WO 03/004674),
das für die Glucose-spezifische IIBC Komponente kodierende ptsG-Gen (WO 03/004670),
das für die Cystein-Synthase A kodierende cysK-Gen (WO 03/006666),
das für den Regulator des cys-Regulons kodierende cysB-Gen (WO 03/006666),
das für das Flavoprotein der NADPH-Sulfit-Reduktase kodierende cysJ-Gen des cysJIH-Operons (WO 03/006666),
das für das Hämoprotein der NADPH-Sulfit-Reduktase kodierende cysI-Gen des cysJIH-Operons (WO 03/006666),
das für die Adenylylsulfat-Reduktase kodierende cysH-Gen des cysJIH-Operons (WO 03/006666),
das für die Decarboxylase Untereinheit der 2-Ketoglutarat Dehydrogenase kodierende sucA-Gen des sucABCD-Operons (WO 03/008614),
das für die Dihydrolipoyltranssuccinase E2 Untereinheit der 2-Ketoglutarat Dehydrogenase kodierende sucB-Gen des sucABCD-Operons (WO 03/008614),
das für die β-Untereinheit der Succinyl-CoA Synthetase kodierende sucC-Gen des suc ABCD-Operons (WO 03/008615),
das für die α-Untereinheit der Succinyl-CoA Synthetase kodierende sucD-Gen des sucABCD-Operons (WO 03/008615),
das Genprodukt des offenen Leserahmens (ORF) yjcG von Escherichia coli (Accession Number NC000913 (Region 4281276-4282925) des National Center for Biotechnology Information (NCBI, Bethesda, MD, USA), der besseren Übersichtlichkeit halber ist die Sequenz des yjcG-ORFs unter der SEQ ID No. 11 und die des von diesem Leserahmen kodierten Proteins als SEQ ID No. 12 dargestellt),,
das Genprodukt des offenen Leserahmens (ORF) yibD von Escherichia coli (Accession Number AE000439 des National Center for Biotechnology Information (NCBI, Bethesda, MD, USA), der besseren Übersichtlichkeit halber ist die Sequenz des yibD-ORFs unter der SEQ ID No. 13 und die des von diesem Leserahmen kodierten Proteins als SEQ ID No. 14 dargestellt),
das Genprodukt des offenen Leserahmens (ORF) yaaU von Escherichia coli (Accession Number NC000913 (Region 45807-47138) des National Center for Biotechnology Information (NCBI, Bethesda, MD, USA), der besseren Übersichtlichkeit halber ist die Sequenz des yaaU-ORFs unter der SEQ ID No. 15 und die des von diesem Leserahmen kodierten Proteins als SEQ ID No. 16 dargestellt),
das für die Glykogen Synthase kodierende glgA-Gen (Accession Number NC000913 (Region 3,564,623 <-3,566,056) des National Center for Biotechnology Information (NCBI, Bethesda, MD, USA)),
das für die Glukose-1-Phosphat Adenylyltransferase kodierende glgC-Gen (Accession Number NC000913 (Region 3,566,056 <- 3,567,351) des National Center for Biotechnology Information (NCBI, Bethesda, MD, USA)),
das für die Glykogen Phosphorylase kodierende glgP-Gen (Accession Number NC000913 (Region 3,562,157 <-3,564,604) des National Center for Biotechnology Information (NCBI, Bethesda, MD, USA)),
das für das 1,4-α-Glukan verzweigende Enzym kodierende glgB-Gen (Accession Number NC000913 (Region 3,569,339 <-3,571,525) des National Center for Biotechnology Information (NCBI, Bethesda, MD, USA)),
das für die Glykogen Phosphorylase-α-1,6-Glukohydrolase kodierende glgX-Gen (Accession Number NC000913 (Region 3,567,369 <- 3,569,342) des National Center for Biotechnology Information (NCBI, Bethesda, MD, USA)),
verstärkt, insbesondere überexprimiert werden.

Desweiteren können beispielsweise für die Produktion von L-Tryptophan, gleichzeitig eines oder mehrere der Gene, ausgewählt aus der Gruppe
mindestens ein Gen des für die Anthranilat-Synthase, die Antranilat-Phosphoribosyltransferase, die Phosphoribosylanthranilat-Isomerase, die Indol-3-Glyzerinphosphat-Synthase und die Tryptophan-Synthase kodierenden Tryptophan-Operons (Applied and Environmental Microbiology 38(2): 181-190 (1979)),
das für die 3-Phosphoglycerat-Dehydrogenase kodierende serA-Gen (WO87/01130)
das für die Phosphoserinphosphatase kodierende serB-Gen (WO87/01130)
das für die Phosphoserinaminotransferase kodierende serC-Gen (WO87/01130)
das für die L-Tyrosin sensitive DHAP-Synthase kodierende aroF-Gen (WO87/01130; EP1270721)
das für die L-Phenylalanin sensitive DHAP-Synthase kodierende aroG-Gen (WO87/01130; EP1270721)
das für die L-Tryptophan sensitive DHAP-Synthase kodierende aroH-Gen (WO87/01130; EP1270721)
das für die Phosphoenolpyruvat-Synthase kodierende pps-Gen (WO96/08567)
das für die Phosphoenolpyruvat-Carboxykinase kodierende pckA-Gen (W02004/090125),
das für die Transketolase A kodierende tktA-Gen (US5, 168, 056),
das für die Transketolase B kodierende tktB-Gen (US5, 168, 056),
das Genprodukt des offenen Leserahmens (ORF) yddG von Escherichia coli (Accession Number NC000913 (Region 1544312-1545193) des National Center for Biotechnology Information (NCBI, Bethesda, MD, USA)),
das für die Glykogen Synthase kodierende glgA-Gen (Accession Number NC000913 (Region 3,564,623 <-3,566,056) des National Center for Biotechnology Information (NCBI, Bethesda, MD, USA)),
das für die Glukose-1-Phosphat Adenylyltransferase kodierende glgC-Gen (Accession Number NC000913 (Region 3,566,056 <- 3,567,351) des National Center for Biotechnology Information (NCBI, Bethesda, MD, USA)),
das für die Glykogen Phosphorylase kodierende glgP-Gen (Accession Number NC000913 (Region 3,562,157 <-3,564,604) des National Center for Biotechnology Information (NCBI, Bethesda, MD, USA)),
das für das 1,4-α-Glukan verzweigende Enzym kodierende glgB-Gen (Accession Number NC000913 (Region 3,569,339 <-3,571,525) des National Center for Biotechnology Information (NCBI, Bethesda, MD, USA)),
das für die Glykogen Phosphorylase-α-1,6-Glukohydrolase kodierende glgX-Gen (Accession Number NC000913 (Region 3,567,369 <- 3,569,342) des National Center for Biotechnology Information (NCBI, Bethesda, MD, USA)),
verstärkt, insbesondere überexprimiert werden.

L-Threonin produzierende Mikroorganismen der Familie Enterobacteriaceae besitzen typischerweise eine "feed back" resistente bzw. desensibilisierte Aspartatkinase I/Homoserin-Dehydrogenase I. Unter "feed back" resistenter Aspartatkinase/Homoserin-Dehydrogenase versteht man Aspartatkinase/Homoserin-Dehydrogenase Enzyme (kodiert durch thrA, EC:2.7.2.41.1.1.3), die im Vergleich zur Wildform eine geringere Empfindlichkeit gegenüber der Hemmung durch Threonin oder Mischungen von Threonin und dem Threonin-Analogon α-Amino-β-Hydroxyvaleriansäure (AHV) oder AHV allein aufweisen. Die für diese desensibilisierten Enzyme kodierenden Gene bzw. Allele werden auch als thrA^{FBR}-Allele bezeichnet. Im Stand der Technik sind thrA^{FBR}-Allele beschrieben, die für Aspartatkinase/Homoserin-Dehydrogenase Varianten kodieren, welche Aminosäureaustausche im Vergleich zum Wildtypprotein besitzen.

Die Kodierregion des Wildtyp thrA-Gens von Escherichia coli entsprechend der Zugangsnummer U00096.2 der NCBI Datenbank (Bethesda, MD, USA) ist in SEQ ID NO:7 und das von diesem Gen kodierte Polypeptid in SEQ ID NO:8 dargestellt.

Auch die Nukleotidsequenz des thrA-Gens von Serratia marcescens ist bekannt und unter der Zugangsnummer X60821 beim NCBI verfügbar. Die Kodierregion des Wildtyp thrA-Gens von Serratia marcescens ist in SEQ ID NO:9 und das von diesem Gen kodierte Polypeptid in SEQ ID NO:10 dargestellt.

Die für die Maßnahmen der Erfindung eingesetzten L-Threonin produzierenden Mikroorganismen der Familie Enterobacteriaceae verfügen bevorzugt über ein thrA-Allel, das für eine Aspartatkinase/Homoserin-Dehydrogenase Variante kodiert, welche die Aminosäuresequenz von SEQ ID NO:8 oder SEQ ID NO:10 besitzt, wobei diese eine oder mehrere der Aminosäureaustausche ausgewählt aus der Gruppe:
ThrA E253K (Austausch von L-Glutaminsäure an Position 253 des kodierten Enzyms Aspartatkinase/Homoserin-Dehydrogenase gemäß SEQ ID NO:8 oder SEQ ID NO:10 gegen L-Lysin; siehe Research Disclosure 505, 537 (2006)),
ThrA G330D (Austausch von Glycin an Position 330 des kodierten Enzyms Aspartatkinase/Homoserin-Dehydrogenase gemäß SEQ ID NO:8 oder SEQ ID NO:10 gegen L-Asparaginsäure; siehe Omori et al. (Journal of Bacteriology 175(3), 785-794 (1993)),
ThrA S345F (Austausch von L-Serin an Position 345 des kodierten Enzyms Aspartatkinase/Homoserin-Dehydrogenase gemäß SEQ ID NO:8 oder SEQ ID NO:10 gegen L-Phenylalanin; siehe Lee et al., Journal of Bacteriology 185(18): 5442-5451 (2003)),
ThrA S352, Austausch von L-Serin an Position 352 des kodierten Enzyms Aspartatkinase/Homoserin-Dehydrogenase gemäß SEQ ID NO:8 oder SEQ ID NO:10 gegen L-Phenylalanin, L-Tyrosin, L-Asparagin, L-Alanin, L-Arginin, L-Glutamin, L-Glutaminsäure, L-Histidin, L-Leucin, L-Methionin, L-Tryptophan oder L-Valin, bevorzugt gegen L-Phenylalanin; siehe Omori et al. (Journal of Bacteriology 175(3), 785-794 (1993) und Omori et al. (Journal of Bacteriology 175(4), 959-965 (1993),
ThrA A479T (Austausch von L-Alanin an Position 479 des kodierten Enzyms Aspartatkinase/Homoserin-Dehydrogenase gemäß SEQ ID NO:8 oder SEQ ID NO:10 gegen L-Threonin; siehe Omori et al. (Journal of Bacteriology 175(3), 785-794 (1993)),
umfasst.

Bevorzugt wird eines der thrA^{FBR}-Allele thrA E253K (Austausch von L-Glutaminsäure an Position 253 des kodierten Enzyms Aspartatkinase/Homoserin-Dehydrogenase gegen L-Lysin)oder S345F (Austausch von L-Serin an Position 345 des kodierten Enzyms Aspartatkinase/Homoserin-Dehydrogenase gegen L-Phenylalanin) gemäß SEQ ID NO:8.

Die hier beschriebenen thrA^{FBR}-Allele, die für ein Aspartatkinase/Homoserin-Dehydrogenase Enzym kodieren, können mit den oben beschriebenen Maßnahmen überexprimiert werden.

Weiterhin kann es für die erhöhte Produktion von L-Aminosäuren, insbesondere L-Threonin, L-Tryptophan, L-Lysin, L-Valin, L-Isoleucin und L-Homoserin vorteilhaft sein, zusätzlich zur Verstärkung des glgS-Gens, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

So können beispielsweise für die Produktion von L-Threonin gleichzeitig eines oder mehrere der Gene, ausgewählt aus der Gruppe
das für die Threonin-Dehydrogenase kodierende tdh-Gen (Journal of Bacteriology 169: 4716-4721 (1987)),
das für die Malat-Dehydrogenase (E.C. 1.1.1.37) kodierende mdh-Gen (Archives in Microbiology 149: 36-42 (1987)),
das für das Enzym Phosphoenolpyruvat-Carboxykinase kodierende pckA-Gen (WO 02/29080),
das für die Pyruvat-Oxidase kodierende poxB-Gen (WO 02/36797),
das für den DgsA-Regulator des Phosphotransferase-Systems kodierende dgsA-Gen (WO 02/081721), das auch unter der Bezeichnung mlc-Gen bekannt ist,
das für den Fructose-Repressor kodierende fruR-Gen (WO 02/081698), das auch unter der Bezeichnung cra-Gen bekannt ist,
das für den Sigma³⁸-Faktor kodierende rpoS-Gen (WO 01/05939), das auch unter der Bezeichnung katF-Gen bekannt ist und
das für die Aspartat Ammonium-Lyase kodierende aspA-Gen (WO 03/008603)
   abgeschwächt, insbesondere ausgeschaltet oder die Expression verringert werden. Diese Massnahmen werden gegebenenfalls zusätzlich zu bzw. in geeigneter Kombination mit den angegebenen Massnahmen zur Erhöhung der Threonin-Produktion durchgeführt.

Weiterhin kann es für die Produktion von L-Tryptophan vorteilhaft sein, zusätzlich zur Verstärkung des glgS-Gens, eines oder mehrere der Gene ausgewählt aus der Gruppe
das für die Tryptophanase kodierende tnaA-Gen (US4,371,614),
das für den Repressor des trp-Operons kodierende trpR-Gen (US4,371,614)
das für die Chorismat-Mutase T und Prephenat-Dehydrogenase kodierende tyrA-Gen (US4,371,614),
das für die Chorismat-Mutase P und Prephenat-Dehydrogenase kodierende pheA-Gen (US4,371,614),
das für das Tryptophan spezifische Transportprotein kodierende mtr-Gen (US5,756,345),
das für die Tryptophan-Permease kodierende tnaB-Gen (US5, 756, 345),
das für den Transporter für aromatische Aminosäuren kodierende aroP-Gen (US5,756,345),
das für die L-Serin Deaminase kodierende sdaA-Gen (EP0149539),
das für die Glukose-6-Phosphat-Isomerase kodierende pgi-Gen (WO87/01130),
das für die Tyrosin-Aminotransferase kodierende tyrB-Gen (WO87/01130)
   abzuschwächen, insbesondere auszuschalten oder die Expression zu verringern. Diese Massnahmen werden gegebenenfalls zusätzlich zu bzw. in geeigneter Kombination mit den angegebenen Massnahmen zur Erhöhung der Tryptophan-Produktion durchgeführt.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfindungsgemäße Abschwächung oder Ausschaltung durchgeführt wird.

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)), Kawano et al. (Nucleic Acids Research 33(19), 6268-6276 (2005)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservativen Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität. Die Verringerung der Genexpression durch Suppression einer Stop-Kodon-Mutation im Kodierbereich durch geeignete t-RNA-Suppressoren ist in der WO 03/074719 beschrieben.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Gegebenenfalls können die Maßnahmen zur Verstärkung und zur Abschwächung beliebig kombiniert werden.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben erhöht.

Erfindungsgemäß werden die hergestellten Mikroorganismen im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert. Zusammenfassungen über derartige Verfahren sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Bei einem batch-Verfahren werden, mit wenigen Ausnahmen, wie beispielsweise Sauerstoff und pH-Korrekturmittel, sämtliche Einsatzstoffe in Form eines Ansatzes vorgelegt und der Mikrorganismus in dem erhaltenen Medium kultiviert.

Bei einem fed batch-Verfahren wird der Mikrorganismus zunächst mittels eines batch-Verfahrens kultiviert (batch-Phase). Im Anschluss daran wird der Kultur ein für die Herstellung des Produktes wesentlicher Einsatzstoff, ggf. auch mehrere Einsatzstoffe, kontinuierlich oder diskontinuierlich hinzugefügt (Zulaufphase, feed-Phase). Im Falle der Herstellung von L-Aminosäuren handelt es sich hierbei um eine Kohlenstoffquelle.

Bei einem repeated fed batch-Verfahren handelt es sich um ein fed batch-Verfahren, bei dem nach Abschluss der Fermentation ein Teil der erhaltenen Fermentationsbrühe als Inokulum zum Start eines erneuten repeated fed batch-Verfahrens dient. Dieser Zyklus kann ggf. mehrfach wiederholt werden. Repeated fed batch-Verfahren sind beispielsweise in der WO 02/18543 und WO 05/014843 beschrieben.

Bei einem kontinuierlichen Verfahren werden im Anschluß an ein batch- oder fed batch-Verfahren ein oder mehrere ggf. sämtliche Einsatzstoffe zu der Kultur kontinuierlich hinzugefügt und gleichzeitig Fermentationsbrühe entnommen. Kontinuierliche Verfahren sind beispielsweise in den Patentschriften US 5,763,230, WO 05/014840, WO 05/014841 und WO 05/014842 beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium, Fermentationsmedium und Nährmedium bzw. Medium sind gegenseitig austauschbar.

Im Allgemeinen enthält ein Kulturmedium unter anderem eine oder mehrere Kohlenstoffquelle(n), Stickstoffquelle(n) und Phosphorquelle(n).

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung bzw. Akkumulation der L-Aminosäure in der Fermentations- bzw. Kulturbrühe. Die Kultur wird solange fortgesetzt, bis sich ein Maximum an der gewünschten L-Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Unter einer Fermentationsbrühe bzw. Kulturbrühe versteht man ein Fermentationsmedium, in dem ein Mikroorganismus für eine gewisse Zeit und bei einer gewissen Temperatur kultiviert wurde.

Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) die im Laufe der Fermentation gebildete L-Aminosäure, c) die im Laufe der Fermentation gebildeten organischen Nebenprodukte und d) die durch die Fermentation nicht verbrauchten Inhaltsstoffe des/ der eingesetzten Fermentationsmediums/ Fermentationsmedien bzw. Anteile der Einsatzstoffe wie beispielsweise Vitamine wie Thiamin oder Salze wie Magnesiumsulfat. Unter den Punkten a, c und d sind die "weiteren" Bestandteile der Fermentationsbrühe benannt.

Die hergestellte Kultur- bzw. Fermentationsbrühe kann anschließend gesammelt und die gewünschte L-Aminosäure bzw. das L-Aminosäure-haltige Produkt gewonnen oder isoliert werden.

In einer Verfahrensvariante wird die Fermentationsbrühe gegebenenfalls konzentriert und anschließend die L-Aminosäure gereinigt bzw. in reiner oder nahezu reiner Form isoliert. Typische Methoden zur Reinigung von L-Aminosäuren sind die Ionenaustauschchromatographie, die Kristallisation, Extraktionsverfahren und die Behandlung mit Aktivkohle. Hierdurch erhält man weitgehend reine L-Aminosäuren mit einem Gehalt von ≥ 90 Gew.-%, ≥ 95 Gew.-%, ≥ 96 Gew.-%, ≥ 97 Gen.-% ≥ 98 Gew.-% oder ≥ 99 Gew.-%.

Es ist ebenfalls möglich in einer anderen Verfahrensvariante aus der hergestellten Fermentationsbrühe ein Produkt herzustellen, indem man die in der Fermentationsbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥60%, ≥70%, ≥80%, ≥90% oder ≥95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10 Gew.-%, kleiner als Gew.-5%, kleiner als Gew.-4% oder kleiner 3 Gew.-% beträgt.

Die Analyse von L-Aminosäuren zur Bestimmung der Konzentration zu einem oder mehreren Zeitpunkt(en) im Verlauf der Fermentation kann durch Anionenaustauschchromatographie mit anschließender Ninhydrin Derivatisierung erfolgen, so wie bei Spackman et al. (Analytical Chemistry 30: 1190-1206 (1958)) beschrieben, oder sie kann durch reversed phase HPLC erfolgen, so wie bei Lindroth et al. (Analytical Chemistry 51: 1167-1174 (1979)) beschrieben.

Das erfindungsgemäße Verfahren dient zur fermentativen Herstellung von L-Aminosäuren, wie beispielsweise L-Threonin, L-Isoleucin, L-Valin, L-Methionin, L-Homoserin, L-Tryptophan und L-Lysin, insbesondere L-Threonin, L-Tryptophan, L-Lysin, L-Valin, L-Isoleucin und L-Homoserin.

Die vorliegende Erfindung wird im Folgenden anhand von Ausführungsbeispielen näher erläutert.

Verwendete Minimal- (M9) und Vollmedien (LB) für Escherichia coli sind von J.H. Miller (A Short Course in Bacterial Genetics (1992), Cold Spring Harbor Laboratory Press) beschrieben. Die Isolierung von Plasmid-DNA aus Escherichia coli sowie alle Techniken zur Restriktion, Ligation, Klenow- und alkalische Phosphatasebehandlung werden nach Sambrook et al. (Molecular Cloning - A Laboratory Manual (1989) Cold Spring Harbor Laboratory Press) durchgeführt. Die Transformation von Escherichia coli wird, wenn nicht anders beschrieben, nach Chung et al. (Proceedings of the National Academy of Sciences of the United States of America 86: 2172-2175 (1989)) durchgeführt.

Die Bebrütungstemperatur bei der Herstellung von Stämmen und Transformanten ist 37°C.

### Beispiel 1

Konstruktion des Expressionsplasmides pTrc99AglgS

Das Gen glgS aus E. coli K12 wurde unter Anwendung der Polymerase-Kettenreaktion (PCR) sowie synthetischer Oligonukleotiden amplifiziert. Ausgehend von der Nukleotidsequenz des glgS-Gens in E. coli K12 MG1655 (Accession Number NC_000913 (Region: complement(3189761-3189961)) wurden PCR-Primer synthetisiert (MWG Biotech, Ebersberg, Deutschland). Die Sequenzen der Primer wurden so modifiziert, dass Erkennungsstellen für Restriktionsenzyme entstanden. Für den glgS1-Primer wurde die Erkennungssequenz für XbaI und für den glgS2-Primer die Erkennungssequenz für HindIII gewählt, die in der unten dargestellten Nukleotidabfolge durch Unterstreichen markiert sind:
glgS1: 5' - GGCCTCTAGA GGTATGCGAT AACCGGAGGA TG - 3'
glgS2: 5' - CGGGCCATCA AGCTTGTTAT ATCAGTGC - 3'

Die für die PCR eingesetzte chromosomale E. coli K12 MG1655 DNA wurde nach Herstellerangaben mit "Qiagen Genomic-tips 100/G" (QIAGEN, Hilden, Deutschland) isoliert. Ein ca. 260 bp großes DNA-Fragment konnte mit den spezifischen Primern unter Standard-PCR-Bedingungen (Innis et al. (1990) PCR Protocols. A Guide to Methods and Applications, Academic Press) mit der Vent-DNA-Polymerase (New England Biolabs, Frankfurt, Deutschland) amplifiziert werden.

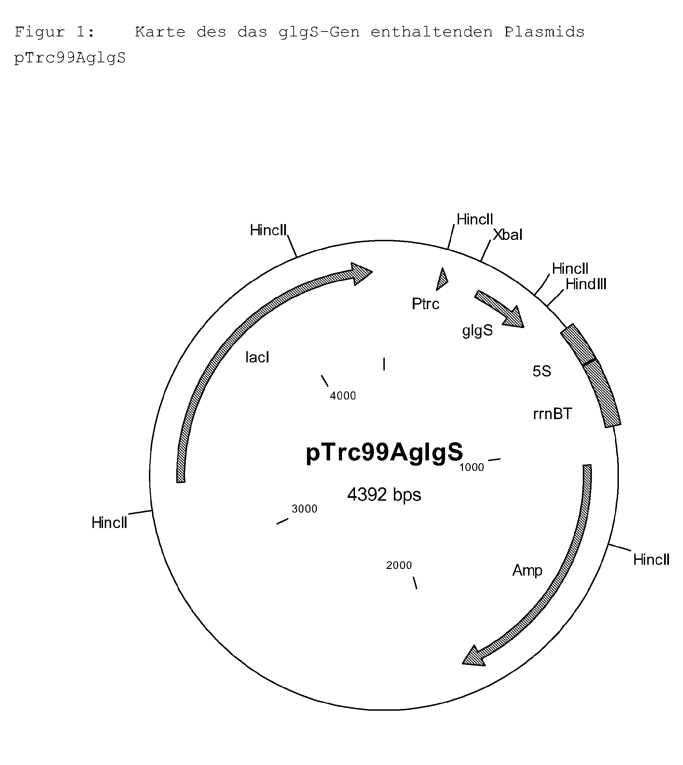

Das PCR-Produkt (die Aminosäuresequenz des korrespondierenden Proteins entspricht SEQ ID No. 2) wurde mit den Restriktionsenzymen XbaI und HindIII gespalten und mit dem Vektor pTrc99A (Pharmacia Biotech, Uppsala, Schweden), der mit den Enzymen XbaI und HindIII verdaut worden ist, ligiert. Der E. coli Stamm TOP10 (Invitrogen, Paisley, UK) wurde mit dem Ligationsansatz transformiert und Plasmid tragende Zellen auf LB Agar, der mit 50 µg/ml Ampicillin versetzt ist, selektioniert. Die erfolgreiche Klonierung konnte nach der Plasmid DNA Isolierung durch die Kontrollspaltung mit den Enzymen HincI, HindIII und XbaI nachgewiesen werden. Das Plasmid wird als pTrc99AglgS (Figur 1) bezeichnet.

### Beispiel 2

Herstellung von L-Threonin mit dem Stamm MG442/pTrc99AglgS Der L-Threonin produzierende E. coli Stamm MG442 ist in der Patentschrift US-A- 4,278,765 beschrieben und bei der Russischen Nationalsammlung für industrielle Mikroorganismen (VKPM, Moskau, Russland) als CMIM B-1628 hinterlegt.

Der Stamm MG442 wurde mit dem in Beispiel 1 beschriebenen Expressionsplasmid pTrc99AglgS und mit dem Vektor pTrc99A transformiert und auf LB Agar mit 50 µg/ml Ampicillin Plasmid tragende Zellen selektioniert. Auf diese Weise entstehen die Stämme MG442/pTrc99AglgS und MG442/pTrc99A. Ausgewählte Einzelkolonien wurden anschließend auf Minimalmedium mit der folgenden Zusammensetzung: 3,5 g/l Na₂HPO₄*2H₂O, 1,5 g/l KH₂PO₄, 1 g/l NH₄Cl, 0,1 g/l MgSO₄*7H₂O, 2 g/l Glucose, 20 g/l Agar, 50 mg/l Ampicillin, weiter vermehrt. Die Bildung von L-Threonin wurde in batch Kulturen von 10 ml, die in 100 ml Erlenmeierkolben enthalten sind, überprüft. Dazu wurde 10 ml Vorkulturmedium der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glucose, 50 mg/l Ampicillin, beimpft und für 16 Stunden bei 37°C und 180 rpm auf einem ESR Inkubator der Firma Kühner AG (Birsfelden, Schweiz) inkubiert.

Je 250 µl dieser Vorkultur wurden in 10 ml Produktionsmedium (25 g/l (NH₄)₂SO₄, 2 g/l KH₂PO₄, 1 g/l MgSO₄*7H₂O, 0,03 g/l FeSO₄*7H₂O, 0,018 g/l MnSO₄*1H₂O, 30 g/l CaCO₃, 20 g/l Glucose, 50 mg/l Ampicillin) überimpft und für 48 Stunden bei 37°C inkubiert. Die Bildung von L-Threonin durch den Ausgangsstamm MG442 wird in der gleichen Weise überprüft, wobei jedoch keine Zugabe von Ampicillin zum Medium erfolgt. Nach der Inkubation wurde die optische Dichte (OD) der Kultursuspension mit einem LP2W-Photometer der Firma Dr. Lange (Düsseldorf, Deutschland) bei einer Messwellenlänge von 660 nm bestimmt.

Anschließend wurde die Konzentration an gebildetem L-Threonin im steril filtrierten Kulturüberstand mit einem Aminosäureanalysator der Firma Eppendorf-BioTronik (Hamburg, Deutschland) durch Ionenaustauschchromatographie und Nachsäulenreaktion mit Ninhydrindetektion bestimmt.

In Tabelle 1 ist das Ergebnis des Versuches dargestellt.

**Tabelle 1**

| Stamm | OD (660 nm) | L-Threonin g/l |
|---|---|---|
| MG442 | 5,6 | 1,4 |
| MG442/pTrc99A | 3,0 | 1,3 |
| MG442/pTrc99AglgS | 5,5 | 2,2 |

### Kurze Beschreibung der Figur:

- Figur 1:: Karte des das glgS-Gen enthaltenden Plasmids pTrc99AglgS

Längenangaben sind als ca.-Angaben aufzufassen. Die verwendeten Abkürzungen und Bezeichnungen haben folgende Bedeutung:

| | |
|---|---|
| Amp: | Ampicillinresistenzgen |
| lacI: | Gen für das Repressorprotein des trc-Promotors |
| Ptrc: | trc-Promotorregion, IPTG-induzierbar |
| glgS: | Kodierregion des glgS-Gens |
| 5S: | 5S rRNA-Region |
| rrnBT: | rRNA-Terminator-Region |

Die Abkürzungen für die Restriktionsenzyme haben folgende Bedeutung
HincII: Restriktionsendonuklease aus Haemophilus influenzae Rc
HindIII: Restriktionsendonuklease aus Haemophilus influenzae Rd
XbaI: Restriktionsendonuklease aus Xanthomonas campestris

## Patentansprüche

1. Rekombinante L-Aminosäuren produzierende Mikroorganismen der Familie Enterobacteriaceae, die ein verstärktes oder überexprimiertes glgS-Gen enthalten, welches für ein Protein kodiert, das als Regulator in die Glykogen-Biosynthese involviert ist.

2. Mikroorganismen gemäss Anspruch 1, in denen ein dem glgS-Gen entsprechendes Polynukleotid verstärkt wird, das für ein Polypeptid kodiert, dessen Aminosäuresequenz zu mindestens 90% identisch ist mit einer Aminosäuresequenz, ausgewählt aus der Gruppe SEQ ID No. 2, SEQ ID No. 4 und SEQ ID No. 6 .

3. Mikroorganismen gemäss Anspruch 2, **dadurch gekennzeichnet, dass** sie ein für das Protein GlgS kodierendes isoliertes Polynukleotid enthalten ausgewählt aus der Gruppe:
a) Polynukleotid mit einer Nukleotidsequenz, ausgewählt aus SEQ ID No. 1, SEQ ID No. 3 oder SEQ ID No. 5 und den dazu komplementären Nukleotidsequenzen;
b) Polynukleotid mit einer Nukleotidsequenz, die der SEQ ID No. 1, SEQ ID No. 3 oder SEQ ID No. 5 im Rahmen der Degeneration des genetischen Codes entspricht;
c) Polynukleotidsequenz mit einer Sequenz, die mit der zur Sequenz SEQ ID No. 1 oder SEQ ID No. 3 komplementären Sequenz unter stringenten Bedingungen hybridisiert, wobei die stringenten Bedingungen durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1, SEQ ID No. 3 oder SEQ ID No. 5, die funktionsneutrale Sinnmutanten enthält,
wobei die Polynukleotide für ein Protein kodieren, das als Regulator in die Glykogen-Biosynthese involviert ist.

4. Mikroorganismen gemäss Anspruch 2, **dadurch gekennzeichnet, dass** das Polypeptid eine Aminosäuresequenz besitzt, die zu wenigstens 95% identisch ist mit einer der Sequenzen ausgewählt aus der Gruppe SEQ ID No. 2, SEQ ID No. 4 und SEQ ID No. 6.

5. Mikroorganismen gemäss Anspruch 2, **dadurch gekennzeichnet, dass** das Polypeptid die Aminosäuresequenz aufweist, die 100% identisch ist mit einer der Sequenzen, ausgewählt aus der Gruppe SEQ ID No. 2, SEQ ID No. 4 und SEQ ID No. 6.

6. Mikroorganismen gemäss den Ansprüchen 1 bis 5, **dadurch gekennzeichnet, dass** sie durch Transformation, Transduktion, Konjugation oder eine Kombination dieser Methoden hergestellt werden, mit einem Vektor, der das glgS-Gen, ein Allel dieses Gens oder Teile davon und/oder einen Promotor enthält.

7. Mikroorganismen gemäss den Ansprüchen 1 bis 6, in denen die Kopienzahl des glgS-Gens oder der Allele um mindestens 1 erhöht bezogen auf die Kopienzahl des glgS-Gens im für das glgS-Gen nicht rekombinanten Mikroorganismus oder Elternstamm vorliegt.

8. Mikroorganismen gemäss Anspruch 7, **dadurch gekennzeichnet, dass** die Erhöhung der Kopienzahl des glgS-Gens um mindestens 1 durch Integration des Gens oder der Allele in das Chromosom der Mikroorganismen bewirkt wird.

9. Mikroorganismen gemäss Anspruch 7, **dadurch gekennzeichnet, dass** die Erhöhung der Kopienzahl des glgS-Gens um mindestens 1 durch einen extrachromosomal replizierenden Vektor erzielt wird.

10. Mikroorganismen gemäss den Ansprüchen 1 bis 9, **dadurch gekennzeichnet, dass** man zur Erzielung der Verstärkung
a) die Promotor- und Regulationsregion oder die Ribosomenbindungsstelle stromaufwärts des glgS-Gens mutiert, oder
b) Expresssionskassetten oder Promotoren stromaufwärts des glgS-Gens einbaut.

11. Mikroorganismen gemäss den Ansprüchen 1 bis 9, **dadurch gekennzeichnet, dass** die Expression des glgS-Gens unter der Kontrolle eines die Expression des Gens verstärkenden Promotors steht.

12. Mikroorganismen gemäss den Ansprüchen 1 bis 11,
**dadurch gekennzeichnet, dass** durch die Verstärkung des glgS-Gens die Konzentration oder Aktivität des glgS-Genproduktes (Proteins) um mindestens 10% erhöht sind, bezogen auf die Aktivität oder Konzentration des Genproduktes im für das glgS-Gen nicht rekombinanten Mikroorganismus oder Elternstamm.

13. Mikroorganismen gemäss den Ansprüchen 1 bis 12,
**dadurch gekennzeichnet, dass** die Mikroorganismen aus den Gattungen Escherichia, Erwinia, Providencia und Serratia ausgewählt sind.

14. Mikroorganismen gemäss Anspruch 13, **dadurch gekennzeichnet, dass** zusätzlich weitere Gene des Biosyntheseweges der gewünschten L-Aminosäure verstärkt, insbesondere überexprimiert vorliegen.

15. Mikroorganismen gemäss den Ansprüchen 1 bis 14,
**dadurch gekennzeichnet, dass** sie L-Threonin, L-Tryptophan, L-Lysin, L-Valin, L-Isoleucin oder L-Homoserin produzieren.

16. Verfahren zur Herstellung von L-Aminosäuren durch Fermentation von rekombinanten Mikroorganismen der Familie Enterobacteriaceae, **dadurch gekennzeichnet, dass** man
a) die die gewünschte L-Aminosäure produzierenden Mikroorganismen gemäß den Ansprüchen 1 bis 15 in einem Medium kultiviert unter Bedingungen, bei denen die gewünschte L-Aminosäure im Medium oder in den Zellen angereichert wird, und
b) die gewünschte L-Aminosäure isoliert, wobei gegebenenfalls weitere Bestandteile der Fermentationsbrühe und/oder die Biomasse in ihrer Gesamtheit oder Anteilen (≥ 0 bis 100%) im isolierten Produkt verbleiben oder vollständig entfernt werden.

17. Verfahren gemäss Anspruch 16 , **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) mindestens ein Gen des für die Aspartatkinase, die Homoserin-Dehydrogenase, die Homoserinkinase und die Threoninsynthase kodierenden thrABC-Operons,
b) das für die Pyruvat-Carboxylase kodierende pyc-Gen von Corynebacterium glutamicum,
c) das für die Phosphoenolpyruvat-Synthase kodierende pps-Gen,
d) das für die Phosphoenolpyruvat-Carboxylase kodierende ppc-Gen,
e) die für die Untereinheiten der Pryridin-Transhydrogenase kodierenden Gene pntA und pntB,
f) das für das Threoninresistenz vermittelnde Protein kodierende Gen rhtC,
g) das für das Threonin-Export-Carrier-Protein kodierende thrE-Gen aus Corynebacterium glutamicum,
h) das für die Glutamat-Dehydrogenase kodierende gdhA-Gen,
i) das für die Phosphohistidin-Protein-Hexose-Phosphotransferase kodierende ptsH-Gen,
j) das für das Enzym I des Phosphotransferase-Systems kodierende ptsI-Gen,
k) das für die Glucose-spezifische IIA Komponente kodierende crr-Gen,
l) das für die Glucose-spezifische IIBC Komponente kodierende ptsG-Gen,
m) das für die Cystein-Synthase A kodierende cysK-Gen,
n) das für den Regulator des cys-Regulons kodierende cysB-Gen,
o) das für das Flavoprotein der NADPH-Sulfit-Reduktase kodierende cysJ-Gen,
p) das für das Hämoprotein der NADPH-Sulfit-Reduktase kodierende cysI-Gen,
q) das für die Adenylylsulfat-Reduktase kodierende cysH-Gen,
r) das für die Decarboxylase Untereinheit der 2-Ketoglutarat Dehydrogenase kodierende sucA-Gen,
s) das für die Dihydrolipoyltranssuccinase E2 Untereinheit der 2-Ketoglutarat Dehydrogenase kodierende sucB-Gen,
t) das für die β-Untereinheit der Succinyl-CoA Synthetase kodierende sucC-Gen,
u) das für die α-Untereinheit der Succinyl-CoA Synthetase kodierende sucD-Gen,
v) das Genprodukt des offenen Leserahmens (ORF) yjcG von Escherichia coli,
w) das Genprodukt des offenen Leserahmens (ORF) yibD von Escherichia coli,
x) das Genprodukt des offenen Leserahmens (ORF) yaaU von Escherichia coli,
y) das für die Glykogen Synthase kodierende glgA-Gen (Accession Number NC000913 (Region 3,564,623 <-3,566,056) des National Center for Biotechnology Information (NCBI, Bethesda, MD, USA)),
z) das für die Glukose-1-Phosphat Adenylyltransferase kodierende glgC-Gen (Accession Number NC000913 (Region 3,566,056 <- 3,567,351) des National Center for Biotechnology Information (NCBI, Bethesda, MD, USA)),
aa) das für die Glykogen Phosphorylase kodierende glgP-Gen (Accession Number NC000913 (Region 3,562,157 <-3,564,604) des National Center for Biotechnology Information (NCBI, Bethesda, MD, USA)),
bb) das für das 1,4-α-Glukan verzweigende Enzym kodierende glgB-Gen (Accession Number NC000913 (Region 3,569,339 <- 3,571,525) des National Center for Biotechnology Information (NCBI, Bethesda, MD, USA)),
cc) das für die Glykogen Phosphorylase-α-1,6-Glukohydrolase kodierende glgX-Gen (Accession Number NC000913 (Region 3,567,369 <- 3,569,342) des National Center for Biotechnology Information (NCBI, Bethesda, MD, USA)),
verstärkt, insbesondere überexprimiert.

18. Verfahren gemäss den Ansprüchen 1 bis 16, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
a) mindestens ein Gen des für die Anthranilat-Synthase, die Antranilat-Phosphoribosyltransferase, die Phosphoribosylanthranilat-Isomerase, die Indol-3-Glyzerinphosphat-Synthase und die Tryptophan-Synthase kodierenden Tryptophan-Operons,
b) das für die 3-Phosphoglycerat-Dehydrogenase kodierende serA-Gen,
c) das für die Phosphoserinphosphatase kodierende serB-Gen,
d) das für die Phosphoserinaminotransferase kodierende serC-Gen,
e) das für die L-Tyrosin sensitive DHAP-Synthase kodierende aroF-Gen,
f) das für die L-Phenylalanin sensitive DHAP-Synthase kodierende aroG-Gen,
g) das für die L-Tryptophan sensitive DHAP-Synthase kodierende aroH-Gen,
h) das für die Phosphoenolpyruvat-Synthase kodierende pps-Gen,
i) das für die Phosphoenolpyruvat-Carboxykinase kodierende pckA-Gen,
j) das für die Transketolase A kodierende tktA-Gen,
k) das für die Transketolase B kodierende tktB-Gen, und
l) das Genprodukt des offenen Leserahmens (ORF) yddG von Escherichia coli,
m) das für die Glykogen Synthase kodierende glgA-Gen (Accession Number NC000913 (Region 3,564,623 <-3,566,056) des National Center for Biotechnology Information (NCBI, Bethesda, MD, USA)),
n) das für die Glukose-1-Phosphat Adenylyltransferase kodierende glgC-Gen (Accession Number NC000913 (Region 3,566,056 <- 3,567,351) des National Center for Biotechnology Information (NCBI, Bethesda, MD, USA)),
o) das für die Glykogen Phosphorylase kodierende glgP-Gen (Accession Number NC000913 (Region 3,562,157 <-3,564,604) des National Center for Biotechnology Information (NCBI, Bethesda, MD, USA)),
p) das für das 1,4-α-Glukan verzweigende Enzym kodierende glgB-Gen (Accession Number NC000913 (Region 3,569,339 <- 3,571,525) des National Center for Biotechnology Information (NCBI, Bethesda, MD, USA)),
q) das für die Glykogen Phosphorylase-α-1,6-Glukohydrolase kodierende glgX-Gen (Accession Number NC000913 (Region 3,567,369 <- 3,569,342) des National Center for Biotechnology Information (NCBI, Bethesda, MD, USA)),
verstärkt, insbesondere überexprimiert.

19. Verfahren gemäss den Ansprüchen 17 bis 18, **dadurch gekennzeichnet, dass** man Mikroorganismen einsetzt, in denen die Stoffwechselwege zumindest teilweise abgeschwächt sind, die die Bildung der gewünschten L-Aminosäure verringern.

20. Verfahren gemäss den Ansprüchen 1 bis 17 und 19,
**dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) das für die Threonin-Dehydrogenase kodierende tdh-Gen,
b) das für die Malat-Dehydrogenase kodierende mdh-Gen,
c) das für die Phosphoenolpyruvat-Carboxykinase kodierende pckA-Gen,
d) das für die Pyruvat-Oxidase kodierende poxB-Gen,
e) das für den DgsA-Regulator des Phosphotransferase-Systems kodierende dgsA-Gen,
f) das für den Fructose-Repressor kodierende fruR-Gen,
g) das für den Sigma³⁸-Faktor kodierende rpoS-Gen, und
h) das für die Aspartat Ammonium-Lyase kodierende aspA-Gen
abschwächt, insbesondere ausschaltet oder die Expression verringert.

21. Verfahren gemäss den Ansprüchen 1 bis 16 oder 18 bis 19, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
a) das für die Tryptophanase kodierende tnaA-Gen,
b) das für den Repressor des trp-Operons kodierende trpR-Gen,
c) das für die Chorismat-Mutase T und Prephenat-Dehydrogenase kodierende tyrA-Gen,
d) das für die Chorismat-Mutase P und Prephenat-Dehydrogenase kodierende pheA-Gen,
e) das für das Tryptophan spezifische Transportprotein kodierende mtr-Gen,
f) das für die Tryptophan-Permease kodierende tnaB-Gen,
g) das für den Transporter für aromatische Aminosäuren kodierende aroP-Gen,
h) das für die L-Serin Deaminase kodierende sdaA-Gen,
i) das für die Glukose-6-Phosphat-Isomerase kodierende pgi-Gen, und
j) das für die Tyrosin-Aminotransferase kodierende tyrB-Gen
abschwächt, insbesondere ausschaltet oder die Expression verringert.

22. Verfahren gemäss den Ansprüchen 16 und 19, **dadurch gekennzeichnet, dass** man L-Aminosäuren, ausgewählt aus der Gruppe L-Asparagin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin herstellt.

23. Verfahren gemäss den Ansprüchen 16 und 19, **dadurch gekennzeichnet, dass** man L-Aminosäuren, ausgewählt aus der Gruppe L-Threonin, L-Tryptophan, L-Lysin, L-Valin, L-Isoleucin und L-Homoserin herstellt.

24. Verfahren gemäss den Ansprüchen 16 und 19, **dadurch gekennzeichnet, dass** die Mikroorganismen in einem batch - Verfahren kultiviert werden.

25. Verfahren gemäss den Ansprüchen 16 und 19, **dadurch gekennzeichnet, dass** die Mikroorganismen in einem fed batch - Verfahren kultiviert werden.

26. Verfahren gemäss den Ansprüchen 16 und 19, **dadurch gekennzeichnet, dass** die Mikroorganismen in einem repeated fed batch-Verfahren kultiviert werden.

27. Verfahren gemäss den Ansprüchen 16 und 19, **dadurch gekennzeichnet, dass** die Mikroorganismen in einem kontinuierlichen Verfahren kultiviert werden.

28. Verfahren gemäss den Ansprüchen 16 und 19, **dadurch gekennzeichnet, dass** die Konzentration der L-Aminosäure zu einem oder mehreren Zeitpunkt(en) im Verlauf der Fermentation bestimmt wird.
